# EUROPÄISCHE PATENTSCHRIFT

(11) **EP 3 994 121 B1**
(45) Veröffentlichungstag und Bekanntmachung des Hinweises auf die Patenterteilung: **12.06.2024**
(21) Anmeldenummer: 20734584.4
(22) Anmeldetag: 01.07.2020
(51) Int. Cl.: C07C 209/36, C07C 209/84, C07C 211/46

(54) **VERFAHREN ZUR REINIGUNG VON ANILIN**
METHOD FOR THE PURIFICATION OF ANILINE
PROCÉDÉ DE NETTOYAGE D'ANILINE

(30) Priorität: 03.07.2019 EP 19184067; 15.06.2020 EP 20180092
(43) Veröffentlichungstag der Anmeldung: 11.05.2022
(73) Patentinhaber: Covestro Deutschland AG, 51373 Leverkusen (DE)
(72) Erfinder: STEFFENS, Friedhelm, 51373 Leverkusen (DE); KRAUSE, Jonas, 40764 Langenfeld (DE); CHAN, Denise, 40219 Düsseldorf (DE)
(74) Vertreter: Levpat
(86) Internationale Anmeldenummer: PCT/EP2020/068525
(87) Internationale Veröffentlichungsnummer: WO 2021/001424

(56) Entgegenhaltungen:
- EP-A1- 1 845 080
- JP-B2- 3 804 082
- Wikipedia: "Aussalzen", , 14. Oktober 2017 (2017-10-14), XP055651165, Gefunden im Internet: URL:https://de.wikipedia.org/wiki/Aussalze n [gefunden am 2019-12-10]

## Beschreibung

Die vorliegende Erfindung betrifft ein Verfahren zur Reinigung von Anilin, umfassend die Schritte: a) Bereitstellen einer Rohanilinfraktion; b) Extrahieren der Rohanilinfraktion mit einem wässrigen Extraktionsmittel enthaltend, bezogen auf die Gesamtmasse des wässrigen Extraktionsmittels, ein Alkalimetallhydroxid in einem Konzentrationsbereich von 0,009 Massen-% bis 2,05 Massen-% und ein von einem Alkalimetallhydroxid verschiedenes Alkalimetallsalz in einem Konzentrationsbereich von 2,40 Massen-% bis 25,0 Massen-%, wobei nach Phasentrennung eine organische Anilinphase und eine wässrige Aminophenolatphase erhalten werden, und c) Destillieren der organischen Anilinphase aus Schritt b) unter Erhalt eines Stroms gereinigten Anilins, einem leichter als Anilin siedende organische Verunreinigungen enthaltenden gasförmigen Strom und einem höher als Anilin siedende organische Verunreinigungen und Anilin enthaltenden flüssigen Strom.

Anilin ist ein wichtiges Zwischenprodukt z. B. zur Herstellung der Di- und Polyisocyanate der Diphenylmethanreihe (MDI) und wird im großtechnischen Maßstab in der Regel durch katalytische Hydrierung von Nitrobenzol in der Gas- oder Flüssigphase hergestellt (siehe z. B. EP-A-0 696 573, EP-A-0 696 574, EP-A-1 882 681 und WO 2013/139737 A1). Bei dieser Reaktion werden neben dem Zielprodukt Anilin auch Nebenkomponenten wie z. B. Phenol oder Aminophenole gebildet, die vor einer weiteren Anwendung des Anilins in Folgeprozessen entfernt werden müssen. Besonders problematisch ist die Abtrennung von solchen Nebenkomponenten, deren Siedepunkte denen des herzustellenden Zielprodukts sehr ähnlich sind, weil hier der destillative Aufwand erheblich ist. Im Fall der Herstellung von Anilin (Siedepunkt bei Normaldruck 184 °C) stellt insbesondere die Abtrennung von Phenol (Siedepunkt bei Normaldruck 182 °C) eine große Herausforderung für die Destillationstechnik dar, was sich in der Verwendung langer Destillationskolonnen mit großer Trennstufenzahl und hohen Rücklaufverhältnissen und entsprechend hohem Energieaufwand widerspiegelt. Eine Vakuumdestillation zur Entfernung von Phenol aus Anilin ist z. B. in DE-OS-1935363 beschrieben.

Aus diesem Grund hat es nicht an Ansätzen gemangelt, Phenol (und andere phenolische Verbindungen) auf andere Art von Anilin zu trennen, insbesondere durch Überführung in Phenolatsalze durch Reaktion mit geeigneten Basen. Die dabei gebildeten Phenolatsalze lassen sich als nichtflüchtige, gut wasserlösliche Verbindungen wesentlich leichter von Anilin trennen als die phenolischen Verbindungen selbst.

So wird in JP-A-49-035341 ein Verfahren beschrieben, bei dem Anilin mit festen Alkalihydroxiden in einem Festbett in Kontakt gebracht und erst anschließend in die Destillation geleitet wird, bzw. bei dem die Destillation in Gegenwart des festen Alkalihydroxides in Anteilen von 0,1 bis 3 Massen-%, bezogen auf die zu destillierende Anilinmenge, durchgeführt wird. Hierdurch wird die Abtrennung kritischer Komponenten wie der Aminophenole vereinfacht. Nachteilig bei diesem Verfahren ist jedoch der Einsatz von hohen molaren Überschüssen der festen Alkalihydroxide in Bezug auf die zu entfernenden sauren Nebenkomponenten und die Unmöglichkeit der genauen Dosierung der alkalischen Verbindungen. Dies kann einerseits bei Überdosierung zu Korrosionsproblemen, Ausfällungen und hochviskosen Sumpfphasen in der Destillationskolonne und andererseits bei Unterdosierung zu einer unvollständigen Entfernung der kritischen Komponenten führen.

EP-B-1 670 747 beschreibt ein Verfahren zur Abtrennung von Verbindungen mit phenolischen Hydroxygruppen von aromatischen Aminen, wobei dem zu reinigenden Amin vor der Destillation eine Base im molaren Verhältnis von 0,5 : 1 bis zu 10 : 1 bezogen auf die phenolischen Verbindungen, zugesetzt wird, optional in Gegenwart von Polyolen, wie z. B. Polyethylenglykol. Gerade für Anilin, welches in sehr großen Mengen hergestellt wird, ist eine solche Zugabe von Polyolen jedoch wirtschaftlich nachteilig und es besteht die Gefahr, das Produkt mit Polyol(-fragmenten) zu kontaminieren. Ohne Einsatz eines solchen Polyols ist aber mit häufigen Prozessunterbrechungen durch Ausfällung von Salzen zu rechnen.

EP-A-1 845 079 beschreibt ein Verfahren zur Reinigung von Anilin durch Zugabe einer wässrigen Alkalimetallhydroxid-Lösung vor oder während der Destillation, wobei die Probleme durch Feststoffabscheidung, Fouling und/oder starken Viskositätsanstieg bei der Destillation dadurch verhindert werden, dass die Sumpfphase der Destillation partiell ausgeschleust, mit Wasser oder verdünnter Alkalihydroxidlösung gewaschen und die organische gewaschene Phase wieder in die Destillation zurückgeführt wird. Nachteilig ist hier die Notwendigkeit eines zusätzlichen Verfahrensschrittes zur Aufrechterhaltung eines zuverlässigen Betriebs. Darüber hinaus fällt in diesem Verfahren ein zusätzlicher, organisch belasteter Abwasserstrom an, der aufbereitet und entsorgt werden muss.

EP-A-2 028 176 beschreibt ein Verfahren zur Reinigung von aromatischen Aminen, bei dem das nach Abtrennung des Prozesswassers erhaltene rohe Amin mit wässriger Alkalimetallhydroxid-Lösung versetzt und das so erhaltene Verfahrensprodukt destilliert wird. Der Sumpf der Destillationskolonne wird teilweise bis vollständig ausgeschleust und teilweise über zwei seriell oder parallel geschaltete Verdampfer (E¹) und (E²) verdampft. Hierdurch soll mit minimalem apparativem und energetischem Aufwand eine maximale Abreicherung des wertvollen Amins im Sumpf der Destillationskolonne erreicht werden.

Bei den zuvor genannten Verfahren wird das aromatische Amin *in Gegenwart einer Base* destilliert. Bei dieser Verfahrensweise müssen Probleme durch Korrosion, Feststoffabscheidung und/oder Fouling bei der Destillation durch aufwändige und/oder teure Maßnahmen verhindert werden. Außerdem sind solche Verfahren nicht gut für die Reinigung von Rohanilin mit hohen Aminophenolgehalten geeignet, da die bei der Umsetzung mit Base gebildeten Aminophenolate nur zu sehr geringen Anteilen in Anilin löslich sind und daher Feststoffe bilden, die in der folgenden Anilinkolonne zu Verschmutzungen bzw. Ablagerungen führen.

Bekannt sind auch solche Verfahren, die das Phenol und andere phenolische Verbindungen durch eine *Extraktion* mit wässriger Base entfernen. Um auch andere, nicht saure Verunreinigungen zu entfernen, muss das Anilin dennoch zumindest teilweise destilliert werden. Solche Verfahren sind beispielsweise in EP-A-1 845 080, EP-A-2 263 997, JP-A-08-295654 oder auch in EP-A-2 641 892 beschrieben. Von diesen sei EP-A-1 845 080 beispielhaft näher betrachtet. Diese Schrift beschreibt ein Verfahren, bei dem durch die Wahl der Konzentration der zur Extraktion verwendeten Alkalimetallhydroxid-Lösung und der Temperatur sichergestellt wird, dass bei der anschließenden Phasentrennung die wässrige Phase stets die untere Phase ist. Auf diese Weise werden Phasentrennprobleme, insbesondere eine Phasenumkehr während der Extraktion vermieden. Bevorzugte Alkalimetallhydroxid-Konzentrationen liegen zwischen 0,71 und 35 Gewichtsprozent. Aber auch höhere Konzentrationen, wie z. B. 50 Gewichtsprozent, werden beschrieben. Geringere Konzentrationen, z. B. bis zu 0,1 Gewichtsprozent, können auch eingesetzt werden, wenn durch eine hinreichend hohe Temperatur (bis zu 140 °C, bevorzugt bis zu 100 °C, besonders bevorzugt bis zu 95 °C) sichergestellt wird, dass die wässrige Phase stets die untere Phase ist. In den Beispielen wurde die Extraktion jeweils bei 90 °C durchgeführt. Es muss also entweder eine hinreichend hohe Alkalimetallhydroxid-Konzentration oder eine hinreichend hohe Temperatur vorliegen, was beides mit Kosten verbunden ist. Wie die für eine gute Phasentrennung erforderlichen Dichteunterschiede zwischen wässriger Phase und organischer Phase ohne diese Maßnahmen erreicht werden kann, ist der Patentanmeldung nicht zu entnehmen.

Die für die Extraktion verwendete Alkalimetallhydroxid-Lösung kann gemäß der Lehre der EP-A-1 845 080 nach der Extraktion, ggf. nach zusätzlicher Reinigung und/oder Aufkonzentrierung, rezykliert und wieder zur Extraktion verwendet werden. Wie dies konkret ausgestaltet werden kann, insbesondere wie die mit organischen Verunreinigungen (insbesondere Phenolat) beladene Alkalimetallhydroxid-Lösung *in wirtschaftlicher Weise* so gereinigt werden kann, dass eine Rezyklierung problemlos möglich wird, ist der Schrift nicht zu entnehmen. Als Alternative zur Rückführung in die Extraktion wird offenbart, die für die Extraktion verwendete Alkalimetallhydroxid-Lösung, ggf. nach zusätzlicher Reinigung, einem Abwasserstrom zuzuführen, der beispielsweise nach anschließender Aufarbeitung einer Kläranlage zugeführt wird. Hier sind Verluste an Alkalimetallhydroxid unvermeidlich. Zusammenfassend kann daher festgestellt werden, dass diese Patentanmeldung zwar ein Verfahren offenbart, mit dem verlässlich sichergestellt werden kann, dass die wässrige Phase stets die untere Phase ist (Vermeidung von Phasenumkehr), dies jedoch mit dem Nachteil einer hohen Alkalimetallhydroxid-Konzentration oder einer hohen Extraktionstemperatur erkauft werden muss. Ferner fehlt ein praktikables Konzept zur Wiederverwendung überschüssigen Alkalimetallhydroxids.

JP 3804082 B2 beschreibt ein Verfahren zur Reinigung Phenol-haltigen Anilins, in welchem Anilin mit verdünnter Alkalimetallhydroxid-Lösung extrahiert wird. Bevorzugt wird eine Konzentration an Alkalimetallhydroxid in der wässrigen Phase nach Vermischen von Anilin und Alkalimetallhydroxid-Lösung im Bereich von 0,1 bis 0,7 Massen-% eingestellt. Das molare Verhältnis von Alkalimetallhydroxid zu Phenolen liegt bevorzugt im Bereich von 3 bis 500.

Um heutige Reinheitsanforderungen zu erfüllen, werden in der Regel mehrere Extraktionsstufen benötigt wie z. B. in JP-A-2007217405 beschrieben, was den Aufwand weiter erhöht. Außerdem fallen in allen diesen Verfahren große Phenolat-haltige Abwasserströme an, die wiederum sorgfältig aufbereitet und entsorgt werden müssen.

Ganz allgemein mit der Verbesserung der Anilinaufarbeitung befasst sich JP-A-2005 350388. Es wird ein Verfahren beschrieben, bei dem ein Teil des Sumpfes der Anilin-Destillationskolonne aus dieser abgeführt und separat, d. h. in einem vom eigentlichen Verdampfer der Kolonne verschiedenen zweiten Verdampfer, in die Gasphase überfahrt wird. Die so erhaltene Gasphase wird in die Reinanilinkolonne zurückgeführt; nicht verdampfbare Schwersiede-Anteile werden abgetrennt. Nachteilig bei diesem Verfahren ist, dass Leichtsieder und Wasser vor der eigentlichen Anilin-Destillationskolonne in einem apparativ aufwändigen Verfahren in einer Entwässerungs-kolonne separat durch eine zusätzliche Destillation abgetrennt werden müssen.

Schließlich ist in EP-A-1 602 640 ein Verfahren beschrieben, bei dem wässrige Amin-Lösung in zwei in Serie geschalteten Destillationskolonnen aufgereinigt wird. Dabei wird eine der Kolonnen bei 2 bis 20 bar und die andere bei 0,1 bis 10 bar betrieben. Die Kondensationswärme der Brüden, die aus der mit höherem Druck betriebenen Kolonne austreten, werden dabei verwendet, um den Sumpf der mit niedrigerem Druck betriebenen Kolonne zu beheizen. Das Amin fällt als Sumpfprodukt an. Deshalb eignet sich das Verfahren nicht zur Abtrennung von Hochsiedern (wie den Aminophenolen) oder Phenol aus Anilin. Aufgrund des hohen Drucks sind außerdem hohe Sumpftemperaturen erforderlich, was zu einer teilweisen Zersetzung des Produkts und damit zu Ausbeuteverlusten oder Fouling führen kann.

Es bestand daher ein Bedarf an weiteren Verbesserungen auf dem Gebiet der Anilinreinigung. Im Bereich der Abtrennung der phenolischen Verbindungen wurde bisher das Hauptaugenmerk auf Phenol selbst und weniger auf Aminophenole gelegt. Die bekannten Verfahren unter Einsatz einer Base weisen bei der Reinigung von Rohanilinfraktionen mit hohen Aminophenolgehalten die oben geschilderten Nachteile auf. Bei Einsatz einer Base in der Destillation tritt das geschilderte Problem der Feststoffbildung auf. Bei Verzicht auf Base ist die Abtrennung des regelmäßig ebenfalls vorhandenen Phenols schwierig. Bei Einsatz einer alkalischen Extraktion werden große Mengen an Base benötigt. Die Konzentration und/oder die Menge der einzusetzen Basenlösung - meist Natrium- oder Kaliumhydroxid - kann nicht beliebig verringert werden, ohne die Extraktionseffizienz und/oder die sich anschließende Phasentrennung zu beeinträchtigen.

Vollkommen überraschend wurde gefunden, dass sich die genannten Probleme lösen oder zumindest deutlich abschwächen lassen, wenn der Destillation des rohen Anilins eine Basenextraktion vorgeschaltet wird, bei welcher zusätzlich zu einem als basische Komponente eingesetzten Alkalimetallhydroxid *auch noch ein von einem Alkalimetallhydroxid verschiedenes Alkalimetallsalz verwendet und insbesondere die nach Phasentrennung erhaltene wässrige Phase nach Ansäuern einer Rückextraktion mit einem organischen Strom wie einem in der Destillation anfallenden Hochsieder-reichen Sumpfstrom unterworfen wird.*

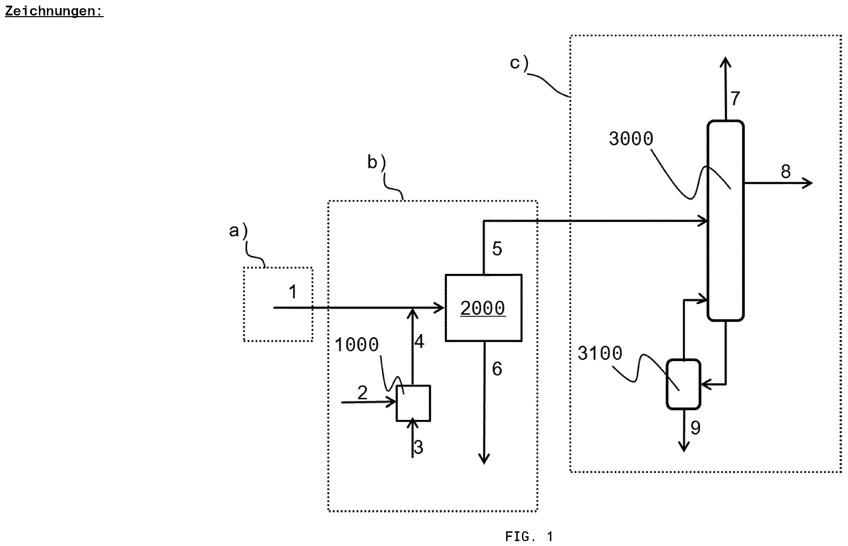

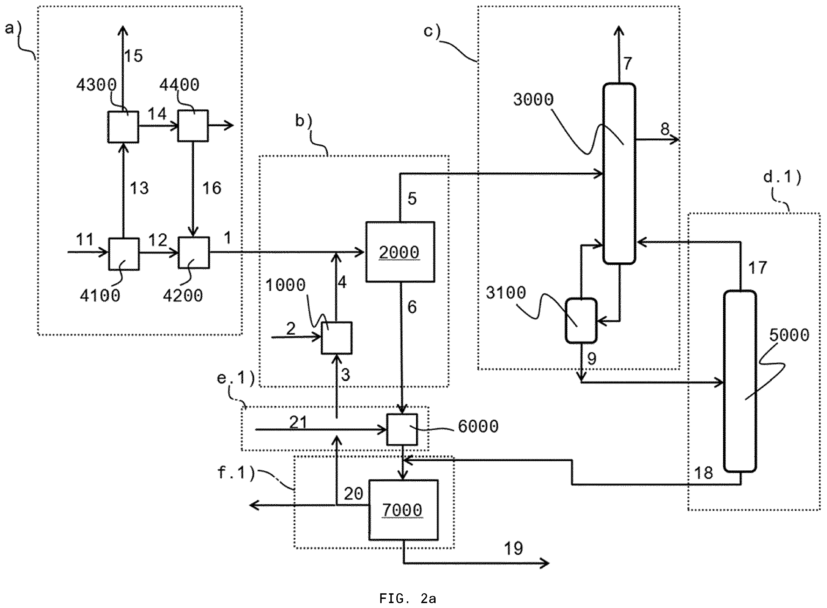

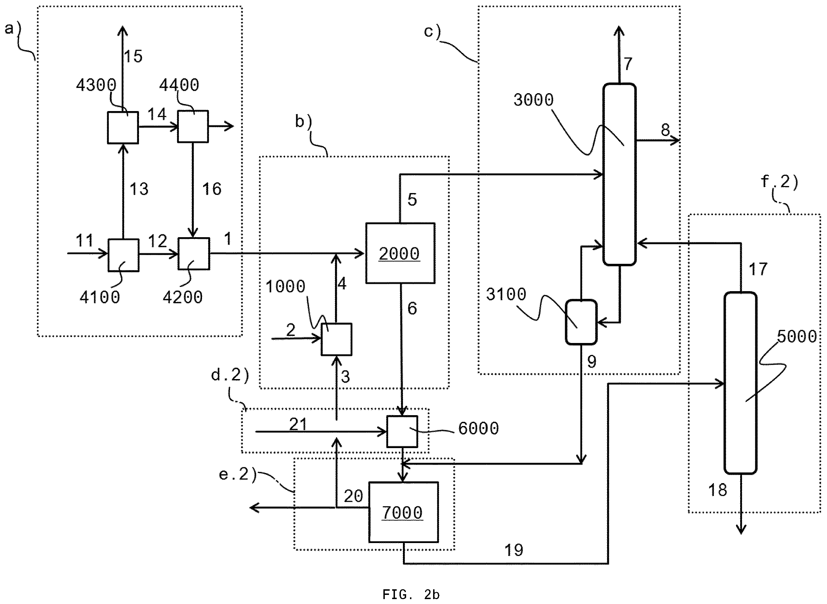

Dem geschilderten Bedarf Rechnung tragend ist daher ein Gegenstand der vorliegenden Erfindung ein Verfahren zur Reinigung von Anilin, umfassend die folgenden Schritte (vgl. auch FIG. 1):
a) Bereitstellen einer (wässrige und organische Bestandteile umfassenden) Rohanilinfraktion (1) enthaltend Anilin und organische Verunreinigungen, wobei die organischen Verunreinigungen Phenol, leichter als Anilin siedende organische Verunreinigungen (sog. "Leichtsieder") und Aminophenol sowie weitere höher als Anilin siedende organische Verunreinigungen (sog. "Hochsieder") umfassen und die auf die Gesamtmasse der organischen Bestandteile der Rohanilinfraktion bezogene Konzentration an Aminophenol im Bereich von 0,001 Massen-% bis 1,00 Massen-%, bevorzugt im Bereich von 0,001 Massen-% bis 0,50 Massen-%, besonders bevorzugt im Bereich von 0,001 Massen-% bis 0,10 Massen-%, liegt;
b) Extrahieren der Rohanilinfraktion aus Schritt a) mit einem wässrigen Extraktionsmittel (4) enthaltend, bezogen auf die Gesamtmasse des wässrigen Extraktionsmittels,
   ein Alkalimetallhydroxid, insbesondere Natrium- oder Kaliumhydroxid, in einem Konzentrationsbereich von 0,009 Massen-% bis 2,05 Massen-%, bevorzugt 0,010 Massen-% bis 2,00 Massen-%, besonders bevorzugt 0,010 Massen-% bis 1,00 Massen-%, ganz besonders bevorzugt 0,50 Massen-% bis 1,00 Massen-%, außerordentlich ganz besonders bevorzugt 0,76 Massen-% bis 1,00 Massen-%,
      und
   ein von einem Alkalimetallhydroxid verschiedenes Alkalimetallsalz, insbesondere Natrium- oder Kaliumchlorid oder Natrium- oder Kaliumsulfat, in einem Konzentrationsbereich von 2,40 Massen-% bis 25,0 Massen-%, bevorzugt 4,00 Massen-% bis 25,0 Massen-%, besonders bevorzugt 5,00 Massen-% bis 25,0 Massen-%, ganz besonders bevorzugt 10,0 Massen-% bis 20,0 Massen-%, außerordentlich ganz besonders bevorzugt 12,0 Massen-% bis 17,0 Massen-%,
   wobei nach Phasentrennung eine organische, an Aminophenol (und selbstverständlich auch an Phenol) abgereicherte, Anilinphase (5) und eine wässrige Aminophenolatphase (6) erhalten werden; und
c) Destillieren der organischen Anilinphase (6) aus Schritt b) unter Erhalt eines Stroms gereinigten Anilins (8), einem leichter als Anilin siedende organische Verunreinigungen enthaltenden gasförmigen Strom (7) und einem höher als Anilin siedende organische Verunreinigungen und Anilin enthaltenden flüssigen Strom (9);
sowie bevorzugt die Schritte d) bis f), umfassend eine Stufe der Aufkonzentrierung, eine Stufe der Säurebehandlung und eine Stufe der Extraktion, wobei es für diese Stufen eine erste Variante (Schritte d.1) bis f.1); vgl. auch FIG. 2a) und eine zweite Variante (Schritte d.2) bis f.2); vgl. auch FIG. 2b) gibt, welche sich in der Reihenfolge der Stufen und der Art der Führung einzelner Stoffströme unterscheiden, wobei die erste Variante umfasst:
d.1) Aufkonzentrieren des höher als Anilin siedende organische Verunreinigungen und Anilin enthaltenden flüssigen Stroms (9) aus Schritt c) durch Verdampfung von Anilin, wobei ein Anilin-haltiger Destillatstrom (17) und ein höher als Anilin siedende organische Verunreinigungen enthaltender Sumpfstrom (18) erhalten werden;
e.1) Behandeln der in Schritt b) erhaltenen wässrigen Aminophenolatphase (6) mit Säure (21) zur Überführung von Aminophenolat in Aminophenol und (bevorzugt parallel zur Zugabe der Säure)
f.1) Extrahieren von durch die Säurebehandlung gebildetem Aminophenol mit einem organischen Strom umfassend den höher als Anilin siedende organische Verunreinigungen enthaltenden Sumpfstrom (18) aus Schritt d.1), wobei nach Phasentrennung eine organische Phase (19) und eine wässrige Phase (20) erhalten werden, wobei die wässrige Phase mit Alkalimetallhydroxid versetzt und als Bestandteil des in Schritt a) einzusetzenden Extraktionsmittels (4) verwendet wird;
und wobei die zweite Variante umfasst:
d.2) Behandeln der in Schritt b) erhaltenen wässrigen Aminophenolatphase (6) mit Säure (21) zur Überführung von Aminophenolat in Aminophenol und (bevorzugt parallel zur Zugabe der Säure)
e.2) Extrahieren von durch die Säurebehandlung gebildetem Aminophenol mit einem organischen Strom umfassend den höher als Anilin siedende organische Verunreinigungen und Anilin enthaltenden flüssigen Strom (9) aus Schritt c), wobei nach Phasentrennung eine organische Phase (19) und eine wässrige Phase (20) erhalten werden, wobei die wässrige Phase mit Alkalimetallhydroxid versetzt und als Bestandteil des in Schritt a) einzusetzenden Extraktionsmittels (4) verwendet wird;
f.2) Aufkonzentrieren der organischen Phase (19) aus Schritt e.2) durch Verdampfung von Anilin, wobei ein Anilin-haltiger Destillatstrom (17) und ein höher als Anilin siedende organische Verunreinigungen enthaltender Sumpfstrom (18) erhalten werden.

Beide bevorzugt durchgeführten Varianten, die erste und die zweite Variante, können mit allen anderen nachfolgend beschriebenen Ausführungsformen der Erfindung kombiniert werden.

Das erfindungsgemäße Verfahren unter Einsatz einer Mischung aus einem Alkalimetallhydroxid und einem von einem Alkalimetallhydroxid verschiedenen Alkalimetallsalz als Extraktionsmittel ermöglicht es, auch bei vergleichsweise geringen Alkalimetallhydroxid-Konzentrationen eine reibungslose Phasentrennung sicherzustellen, ohne (wie etwa in EP-A-1 845 080 gelehrt) auf vergleichsweise hohe Extraktionstemperaturen zurückgreifen zu müssen. Durch die zusätzliche Verwendung eines von einem Alkalimetallhydroxid verschiedenen Alkalimetallsalzes in der Extraktion gemäß Schritt b) wird es ermöglicht, verlässlich zu gewährleisten, dass die wässrige Phase bei der Phasentrennung regelmäßig die untere ("schwere") Phase ist, und zwar ohne bei der Wahl der Temperatur eingeschränkt zu sein und ohne eine vermehrte Verwendung des vergleichsweise teuren Alkalimetallhydroxids. Ferner ist es in bevorzugter Ausgestaltung des erfindungsgemäßen Verfahrens nach einer Ansäuerung möglich, die organischen Bestandteile aus der wässrigen Phase zurückzuextrahieren, was nach Ersatz verbrauchter Base eine Kreislaufführung wesentlicher Bestandteile des Extraktionsmittels möglich macht. Weiterhin ist es möglich und Gegenstand bevorzugter Ausführungsformen, die in der Rückextraktion abgetrennten organischen Bestandteile einem (ohnehin anfallenden) Abfallstrom zuzuführen, was die Entsorgung zusätzlicher Abfallströme überflüssig macht.

In allen technisch relevanten Verfahren zur Herstellung von Anilin wird als Koppelprodukt Wasser (das *Reaktionswasser*) gebildet, sodass in der Herstellung von Anilin regelmäßig zunächst ein zweiphasiges Rohprodukt aus einer wässrigen Phase (enthaltend das Reaktionswasser) und einer organischen Phase (enthaltend das gebildete Anilin und organische Verunreinigungen) erhalten wird. Die nach teilweiser bis vollständiger, bevorzugt vollständiger, Abtrennung der wässrigen Phase aus dem Rohprodukt (durch eine sog., dem Fachmann bekannte, *Phasentrennung*) verbleibende Anilin enthaltende Phase wird im Rahmen der vorliegenden Erfindung als *"Rohanilinfraktion"* bezeichnet. Wie dem Fachmann bekannt ist, ist eine solche Trennung der wässrigen von der organischen Phase eines zweiphasigen Verfahrensproduktes aufgrund einer gewissen Restlöslichkeit der beiden Phasen ineinander nie in dem Sinne perfekt, dass eine restlose Trennung organischer von wässrigen Bestandteilen möglich wäre. Der Begriff *"vollständige Abtrennung der wässrigen Phase"* oder "*vollständige Phasentrennung"* meint daher im Rahmen der vorliegenden Erfindung lediglich, dass Anteile der wässrigen Phase *nicht bewusst* in der organischen Phase belassen werden. Die Rohanilinfraktion umfasst daher selbst bei vollständiger Abtrennung der wässrigen Phase neben den organischen auch wässrige Bestandteile.

Der Begriff *"Aminophenol"* umfasst in der Terminologie der vorliegenden Erfindung alle Isomere (gemeint ist also die Summe aller vorhandenen Isomeren), wobei jedoch erfahrungsgemäß nur ortho- und para-Aminophenol regelmäßig in nachweisbaren Mengen vorhanden sind.

Die im Rahmen der vorliegenden Erfindung angegebenen *Massenkonzentrationen organischer Verbindungen* beziehen sich, sofern es sich um gemessene (und nicht um aus bekannten Einsatzstoffen berechnete) Werte handelt, auf per Gaschromatographie bestimmte Werte. Die Quantifizierung der Konzentration niedermolekularer organischer Verbindungen mittels Gaschromatographie ist ein dem Fachmann wohlbekanntes Verfahren, das an dieser Stelle keiner weiteren Erläuterung bedarf. Bezugsgröße ist im Rahmen der vorliegenden Erfindung die *Gesamtmasse der organischen Bestandteile.*

Die weiter oben bei Schritt b) angegebenen Bereiche und Vorzugsbereiche für die Massenkonzentrationen an *Alkalimetallhydroxid* einerseits und an dem *von einem Alkalimetallhydroxid verschiedenen Alkalimetallsalz* andererseits können grundsätzlich beliebig miteinander kombiniert werden, d. h. es ist beispielsweise möglich, das Alkalimetallhydroxid in einer Konzentration im Bereich von 0,010 Massen-% bis 2,00 Massen-% (zweitbreitester Bereich) und das von einem Alkalimetallhydroxid verschiedene Alkalimetallsalz in einer Konzentration im Bereich von 12,0 Massen-% bis 17,0 Massen-% (engster Bereich) einzusetzen. Dies gilt auch für die einzelnen Unter- bzw. Obergrenzen. Es ist daher beispielsweise ebenfalls möglich, das Alkalimetallhydroxid in einer Konzentration im Bereich von 0,010 Massen-% bis 2,00 Massen-% (zweitbreitester Bereich) und das von einem Alkalimetallhydroxid verschiedene Alkalimetallsalz in einer Konzentration im Bereich von 2,40 Massen-% (Untergrenze des breitesten Bereichs) bis 17,0 Massen-% (Obergrenze des engsten Bereichs) einzusetzen. Besonders bevorzugt ist es jedoch, die einander entsprechenden Bereiche miteinander zu verbinden, also etwa den *bevorzugten* Bereich für die Konzentration des Alkalimetallhydroxids mit dem *bevorzugten* Bereich für die Konzentration des von einem von einem Alkalimetallhydroxid verschiedene Alkalimetallsalz oder den *ganz bevorzugten* Bereich für die Konzentration des Alkalimetallhydroxids mit dem *ganz bevorzugten* Bereich für die Konzentration des von einem von einem Alkalimetallhydroxid verschiedene Alkalimetallsalz usw.

Unter *"Leichtsiedern"* werden im Rahmen der vorliegenden Erfindung solche organischen Verbindungen (und gegebenenfalls Azeotrope) verstanden, die einen geringeren Siedepunkt als Anilin haben. Im Hinblick auf das Siedeverhalten gehört natürlich auch Wasser zu den Leichtsiedern; weil dieses aber keine auf Nebenreaktionen zurückzuführende organische Verunreinigung, sondern entweder ein Koppelprodukt der Anilin-Herstellung ist (Hydrierung von Nitrobenzol, Ammonolyse von Phenol) oder mit den eingesetzten Reagenzien eingeführt wird (Reduktion von Nitrobenzol mit unedlen Metallen wie Eisen in Gegenwart von Salzsäure, Ammonolyse mit wässrigem Ammoniak), wird es im Allgemeinen und auch im Rahmen der vorliegenden Erfindung separat aufgeführt. Typische Leichtsieder im Sinne der vorliegenden Erfindung sind Cyclohexylamin, Cyclohexanon, Cyclohexanol und Benzol. Entsprechend werden unter *"Hochsiedern"* im Rahmen der vorliegenden Erfindung solche organischen Verbindungen (und gegebenenfalls Azeotrope) verstanden, die einen höheren Siedepunkt als Anilin haben. Typische Hochsieder sind Aminophenole, Toluidine, Phenylendiamin, Diphenylamin, N-Cyclohexylanilin oder auch nicht umgesetztes Ausgangsmaterial aus der Herstellung, z. B. Nitrobenzol. Grundsätzlich ist die Einstufung eines Stoffes als Leicht- oder Hochsieder druckabhängig, im vorliegenden Fall also abhängig von den für die Destillation in Schritt c) gewählten Druckbedingungen (ein Stoff kann bei einem bestimmten Druck ein Leichtsieder und bei einem anderen Druck ein Hochsieder sein). Die vorgenannten Beispiele für typische Leicht- und Hochsieder entsprechen jedoch in allen für Schritt c) relevanten Druckbereichen der vorgenommenen Zuordnung. Phenol allerdings weist ein dem Anilin so ähnliches Siedeverhalten auf; das es in Schritt c) abhängig vom vorliegenden Druck als Leicht- oder Hochsieder abgetrennt werden kann.

Unter einem *"Strom gereinigten Anilins"* wird im Rahmen der vorliegenden Erfindung ein Anilinstrom verstanden, der einer destillativen Abtrennung von Leicht- und Hochsiedern unterworfen wurde. Ein erfindungsgemäß erhaltener Strom gereinigten Anilins enthält, auf seine Gesamtmasse bezogen, Anilin in einem Anteil im Bereich von 99,5000 Massen-% bis 99,9999 Massen-%, bevorzugt im Bereich von 99,9000 Massen-% bis 99,9999 Massen-%, besonders bevorzugt im Bereich von 99,9500 Massen-% bis 99,9999 Massen-%.

In den beigefügten Zeichnungen zeigen:
- **FIG. 1**: eine Ausführungsform des erfindungsgemäßen Verfahrens;
- **FIG. 2a**: eine weitere Ausführungsform des erfindungsgemäßen Verfahrens umfassend die weiter oben beschriebene erste Variante;
- **FIG. 2b**: eine weitere Ausführungsform des erfindungsgemäßen Verfahrens umfassend die weiter oben beschriebene zweite Variante;
- **FIG. 3**: den Grad der Abreicherung von Phenol und Aminophenol bei einer Extraktion eines Phenol- und Aminophenol-haltigen Anilins mit verschiedenen Extraktionsmitteln sowie die relative Phasentrennzeit im Vergleich zur Extraktion mit reinem Wasser; und
- **FIG. 4**: die Verteilungskoeffizienten von Phenol und ortho-Aminophenol zwischen organischer und wässrige Phase in einem Zweiphasengemisch bei verschiedenen pH-Werten.

Es folgt zunächst eine Kurzzusammenfassung verschiedener möglicher Ausführungsformen der Erfindung, wobei die Aufzählung der Ausführungsformen nicht als abschließend anzusehen ist.

In einer ersten Ausführungsform der Erfindung, die sowohl mit (siehe die obigen Ausführungen) der ersten Variante als auch mit der zweiten Variante kombinierbar ist, umfasst Schritt c) eine Destillation in einer Destillationskolonne mit Seitenabzug, wobei der Destillationskolonne mit Seitenabzug der Strom gereinigten Anilins als Seitenstrom, der leichter als Anilin siedende organische Verunreinigungen enthaltende gasförmige Strom als Kopfstrom und der höher als Anilin siedende organische Verunreinigungen und Anilin enthaltende flüssige Strom als Sumpfstrom entnommen werden.

In einer zweiten Ausführungsform der Erfindung, die sowohl mit (siehe die obigen Ausführungen) der ersten Variante als auch mit der zweiten Variante kombinierbar ist, umfasst Schritt c) eine Destillation in zwei hintereinandergeschalteten Destillationskolonnen, wobei in der ersten Destillationskolonne der leichter als Anilin siedende organische Verunreinigungen enthaltende gasförmige Strom als Kopfstrom entnommen wird, wobei der in der ersten Destillationskolonne erhaltene Sumpfstrom als Zulauf in die zweite Destillationskolonne geführt wird, wobei in der zweiten Destillationskolonne der Strom gereinigten Anilins als Kopfstrom und der höher als Anilin siedende organische Verunreinigungen und Anilin enthaltende flüssige Strom als Sumpfstrom erhalten werden.

In einer dritten Ausführungsform der Erfindung, die sowohl mit (siehe die obigen Ausführungen) der ersten Variante als auch mit der zweiten Variante kombinierbar ist, umfasst Schritt c) eine Destillation in drei Destillationskolonnen, wobei einer ersten Destillationskolonne ein Wasser und organische Verunreinigungen enthaltener Kopfstrom entnommen wird, der in eine wässrige und eine organische Phase getrennt wird, wobei die organische Phase in eine zweite Destillationskolonne geführt wird, welcher der leichter als Anilin siedende organische Verunreinigungen enthaltende gasförmige Strom als Kopfstrom entnommen wird, und wobei der in der ersten Destillationskolonne erhaltene Sumpfstrom als Zulauf in eine dritte Destillationskolonne geführt wird, wobei in der dritten Destillationskolonne der Strom gereinigten Anilins als Kopfstrom und der höher als Anilin siedende organische Verunreinigungen und Anilin enthaltende flüssige Strom als Sumpfstrom erhalten werden.

In einer vierten Ausführungsform der Erfindung, die eine besondere Ausgestaltung der ersten Ausführungsform bis dritten Ausführungsform ist (also auf jede dieser drei Ausführungsformen angewandt werden kann), wird der leichter als Anilin siedende organische Verunreinigungen enthaltende gasförmige Strom partiell verflüssigt, die so erhaltene flüssige Phase, optional nach Abtrennung von gegebenenfalls vorhandenem Wasser, (als Rücklauf) in diejenige Destillationskolonne, welcher der leichter als Anilin siedende organische Verunreinigungen enthaltende gasförmige Strom als Kopfstrom entnommen wurde, zurückgeführt, wobei die nach der partiellen Verflüssigung verbleibende gasförmige Phase (enthaltend neben nicht kondensierbaren Gasen leichter als Anilin siedende organische Verunreinigungen) aus der Destillation abgeführt wird.

In einer fünften Ausführungsform der Erfindung, die sowohl mit (siehe die obigen Ausführungen) der ersten Variante als auch mit der zweiten Variante kombinierbar ist, wird zur Aufkonzentrierung in Schritt d.1) bzw. in Schritt f.2) eine Destillationskolonne eingesetzt.

In einer sechsten Ausführungsform der Erfindung, die sowohl mit (siehe die obigen Ausführungen) der ersten Variante als auch mit der zweiten Variante kombinierbar ist, wird die in Schritt c) nach der partiellen Verflüssigung verbleibende gasförmige Phase (enthaltend neben nicht kondensierbaren Gasen leichter als Anilin siedende organische Verunreinigungen) kondensiert und das erhaltene Kondensat als zusätzlicher Bestandteil des in Schritt f.1) bzw. Schritt e.2) einzusetzenden organischen Stroms verwendet.

In einer siebten Ausführungsform der Erfindung, die sowohl mit (siehe die obigen Ausführungen) der ersten Variante als auch mit der zweiten Variante kombinierbar ist, wird
- die in Schritt f.1) nach der Phasentrennung erhaltene organische Phase bzw.
- der in Schritt f.2) erhaltene höher als Anilin siedende organische Verunreinigungen enthaltende Sumpfstrom verbrannt.

In einer achten Ausführungsform der Erfindung, die sowohl mit (siehe die obigen Ausführungen) der ersten Variante als auch mit der zweiten Variante kombinierbar ist, wird als das von einem Alkalimetallhydroxid verschiedene Alkalimetallsalz (i) ein Alkalimetallchlorid oder (ii) ein Alkalimetallsulfat eingesetzt.

In einer neunten Ausführungsform der Erfindung, die eine besondere Ausgestaltung der achten Ausführungsform ist, wird in Schritt e.1) bzw. Schritt d.2) als Säure im Fall (i) Salzsäure und im Fall (ii) Schwefelsäure eingesetzt.

In einer zehnten Ausführungsform der Erfindung, die mit allen anderen Ausführungsformen kombiniert werden kann, wird das Extrahieren der Rohanilinfraktion in Schritt b) in mehreren, vorzugsweise in zwei, Stufen im Gegenstrom durchgeführt.

In einer elften Ausführungsform der Erfindung, die mit allen anderen Ausführungsformen kombiniert werden kann, wird die in Schritt b) erhaltene organische Anilinphase vor der Destillation in Schritt c) mit einer wässrigen Lösung eines Alkalimetallhydroxids vermischt und die so erhaltene Mischung ohne vorherige Abtrennung ihrer wässrigen Bestandteile der Destillation aus Schritt c) unterworfen.

In einer zwölften Ausführungsform der Erfindung, die mit allen anderen Ausführungsformen kombiniert werden kann, wird die Rohanilinfraktion durch Hydrierung von Nitrobenzol in Gegenwart eines Katalysators und Abtrennung von in der Hydrierung gebildetem Wasser erhalten.

In einer dreizehnten Ausführungsform der Erfindung, die eine besondere Ausgestaltung der zwölften Ausführungsform ist, wird die Hydrierung von Nitrobenzol in der Gasphase durchgeführt und das erhaltene gasförmige Reaktionsprodukt kondensiert.

In einer vierzehnten Ausführungsform der Erfindung, die eine besondere Ausgestaltung der dreizehnten Ausführungsform ist, wird die Kondensation des gasförmigen Reaktionsprodukts in zwei Stufen (nämlich einer "Partialkondensation" und einer "Totalkondensation") mit sukzessive fallender Temperatur durchgeführt, wobei nur nach der zweiten Kondensationsstufe eine Phasentrennung zur Abtrennung von in der Hydrierung gebildetem Wasser erfolgt.

In einer fünfzehnten Ausführungsform der Erfindung, die eine besondere Ausgestaltung der vierzehnten Ausführungsform ist, wird das in der ersten Kondensationsstufe erhaltene Kondensat mit der nach der Abtrennung von Wasser in der Phasentrennung nach der zweiten Kondensationsstufe verbleibenden organischen Phase vereinigt und (teilweise oder vollständig) als Rohanilinstrom in Schritt a) eingesetzt.

In einer sechzehnten Ausführungsform der Erfindung, die eine weitere besondere Ausgestaltung der vierzehnten Ausführungsform ist, wird der in Schritt a) bereitzustellende Rohanilinstrom *entweder* dem in der ersten Kondensationsstufe erhaltenen Kondensat *oder* der nach der Abtrennung von Wasser in der Phasentrennung nach der zweiten Kondensationsstufe verbleibenden organischen Phase entnommen.

In einer siebzehnten Ausführungsform der Erfindung, die eine weitere besondere Ausgestaltung der dreizehnten Ausführungsform ist, wird die Kondensation des gasförmigen Reaktionsprodukts einstufig durchgeführt, wobei im Anschluss an die Kondensation eine Phasentrennung zur Abtrennung von in der Hydrierung gebildetem Wasser erfolgt, und wobei die nach Abtrennung des Wassers verbleibende organische Phase (teilweise oder vollständig) als Rohanilinstrom in Schritt a) eingesetzt wird.

In einer achtzehnten Ausführungsform der Erfindung, die eine besondere Ausgestaltung der zwölften Ausführungsform bis siebzehnten Ausführungsform ist, wird die Hydrierung adiabatisch durchgeführt.

In einer neunzehnten Ausführungsform der Erfindung, die eine weitere besondere Ausgestaltung der zwölften Ausführungsform bis siebzehnten Ausführungsform ist, wird die Hydrierung isotherm durchgeführt.

In einer zwanzigsten Ausführungsform der Erfindung, die eine besondere Ausgestaltung der achtzehnten Ausführungsform ist, umfasst der Katalysator Kupfer auf einem Siliciumdioxid-Träger.

In einer einundzwanzigsten Ausführungsform der Erfindung, die eine besondere Ausgestaltung der neunzehnten Ausführungsform ist, umfasst der Katalysator Palladium auf einem Aluminiumoxid-Träger.

In einer zweiundzwanzigsten Ausführungsform der Erfindung, die mit allen anderen Ausführungsformen kombiniert werden kann, ist das Alkalimetall des Alkalimetallhydroxids und des von einem Alkalimetallhydroxid verschiedenen Alkalimetallsalzes jeweils Natrium oder Kalium.

In einer dreiundzwanzigsten Ausführungsform der Erfindung, die mit allen anderen Ausführungsformen kombiniert werden kann, wird in Schritt b) ein molares Verhältnis von Alkalimetallhydroxid zu phenolischen Hydroxygruppen im Bereich von 0,80 bis 50, bevorzugt im Bereich von 0,95 bis 10, besonders bevorzugt im Bereich von 1,0 bis 5,0 und ganz besonders bevorzugt im Bereich von 1,0 bis 2,4, eingestellt.

In einer vierundzwanzigsten Ausführungsform der Erfindung, die mit allen anderen Ausführungsformen kombiniert werden kann, wird die Extraktion in Schritt b) bei einer Temperatur im Bereich von 20 °C bis 95 °C, besonders bevorzugt 25 °C bis 85 °C, ganz besonders bevorzugt 25 °C bis 70 °C und außerordentlich ganz besonders bevorzugt 25 °C bis 40 °C durchgeführt.

In einer fünfundzwanzigsten Ausführungsform der Erfindung, die mit allen anderen Ausführungsformen, welche die Schritte d) bis f) (in der ersten oder zweiten Variante) umfassen, kombiniert werden kann, wird das Extrahieren von durch die Säurebehandlung gebildetem Aminophenol mit einem organischen Strom (Schritt f.1 bzw. Schritt e.2) in einer Mixer-Settler-Apparatur (Mischabsetzer) durchgeführt, die eine Mischeinheit und eine Trenneinheit umfasst, wobei das Behandeln der in Schritt b) erhaltenen wässrigen Aminophenolatphase (6) mit Säure (21) zur Überführung von Aminophenolat in Aminophenol derart durchgeführt wird, dass die in Schritt b) erhaltene wässrige Aminophenolatphase (6) in der Mischeinheit mit der Säure (21) und dem organischen Strom (umfassend den Strom 18 bzw. 9) vermischt wird (simultane Zugabe der Säure und des organischen Stroms zur wässrigen Aminophenolatphase, d. h. die Extraktion mit dem organischen Strom wird parallel zur Säurebehandlung durchgeführt), woran sich eine Phasentrennung in eine organische Phase (19) und eine wässrige Phase (20) in der Trenneinheit anschließt.

In einer sechsundzwanzigsten Ausführungsform der Erfindung, die mit allen anderen Ausführungsformen kombiniert werden kann,
wird als Alkalimetallhydroxid
   Natrium- oder Kaliumhydroxid in einem Konzentrationsbereich von 0,009 Massen-% bis 2,05 Massen-%, bevorzugt 0,010 Massen-% bis 2,00 Massen-%, besonders bevorzugt 0,010 Massen-% bis 1,00 Massen-%, ganz besonders bevorzugt 0,50 Massen-% bis 1,00 Massen-%, außerordentlich ganz besonders bevorzugt 0,76 Massen-% bis 1,00 Massen-%,
eingesetzt, und als von einem Alkalimetallhydroxid verschiedenes Alkalimetallsalz wird
   entweder
Natrium- oder Kaliumchlorid in einem Konzentrationsbereich von 4,00 Massen-% bis 25,0 Massen-%, bevorzugt 5,00 Massen-% bis 25,0 Massen-%, besonders bevorzugt 10,0 Massen-% bis 20,0 Massen-%, ganz besonders bevorzugt 12,0 Massen-% bis 17,0 Massen-%,
   oder
Natrium- oder Kaliumsulfat, in einem Konzentrationsbereich von 2,40 Massen-% bis 25,0 Massen-%, bevorzugt 4,00 Massen-% bis 25,0 Massen-%, besonders 5,00 Massen-% bis 25,0 Massen-%, ganz besonders bevorzugt 10,0 Massen-% bis 20,0 Massen-%, außerordentlich ganz besonders bevorzugt 12,0 Massen-% bis 17,0 Massen-%,
   eingesetzt.

Die zuvor kurz geschilderten und weitere mögliche Ausführungsformen der Erfindung werden im Folgenden näher erläutert. Dabei können Ausführungsformen beliebig miteinander kombiniert werden, sofern sich aus dem Zusammenhang nichts anderes ergibt.

**FIG. 1** zeigt eine einfache Ausgestaltung des erfindungsgemäßen Verfahrens. Die Bezugszeichen haben die folgende Bedeutung.

### Apparate bzw. Verfahrensschritte:

| | |
|---|---|
| 1000 | Mischer; |
| 2000 | Basenextraktion inklusive Phasentrennung; |
| 3000 | Destillationskolonne; |
| 3100 | Sumpfverdampfer der Destillationskolonne 3000 |

### Stoffströme :

| | |
|---|---|
| 1 | Rohanilinfraktion; |
| 2 | Alkalimetallhydroxidlösung; |
| 3 | davon verschiedene Alkalimetallsalzslösung (bevorzugt Akalimetallchlorid- oder Alkalimetallsulfatlösung); |
| 4 | wässriges Extraktionsmittel ; |
| 5 | organische, an Aminophenol (und Phenol) abgereicherte, Anilinphase; |
| 6 | Wässrige Aminophenolatphase; |
| 7 | Kopfstrom der Destillationskolonne (Leichtsieder, Wasser, nicht kondensierbare Gase); |
| 8 | gereinigtes Anilin; |
| 9 | Austrag des Sumpfverdampfers; |
| 10 | Destillat des Sumpfverdampfers. |

Die im Stand der Technik derzeit üblichen industriellen Verfahren zur Herstellung von Anilin (insbesondere die Hydrierung von Nitrobenzol, in geringerem Umfang die Reduktion von Nitrobenzol mit unedlen Metallen wie Eisen, sowie - heute nur noch wenig bedeutsam - die Ammonolyse von Phenol oder Chlorbenzol) liefern trotz ihrer Verschiedenheit letztlich alle ein Phenol- und Aminophenol-haltiges Rohanilin, sodass das erfindungsgemäße Verfahren auf alle diese Herstellverfahren sinnvoll anwendbar ist und somit auch das in Schritt a) bereitzustellende Rohanilin aus allen diesen Verfahren stammen kann.

Die eigentliche Herstellung des zu reinigenden Anilins erfolgt bevorzugt durch katalytische Hydrierung von Nitrobenzol. Das dabei erhaltene rohe Verfahrensprodukt enthält neben Anilin und Nebenkomponenten beträchtliche Anteile an Wasser als Koppelprodukt der Hydrierung (Reaktionswasser), welches vor der weiteren Reinigung teilweise bis vollständig, bevorzugt vollständig, abgetrennt wird. Die Abtrennung erfolgt durch dem Fachmann bekannte Phasentrennung. Die dabei erhaltene wässrige Phase enthält in der bevorzugten Ausgestaltung mit vollständiger Phasentrennung (siehe hierzu die Erläuterungen weiter oben) den Hauptteil des Reaktionswassers (bis auf eine Restmenge, die infolge einer geringen Löslichkeit von Wasser in Anilin nicht durch Phasentrennung abgetrennt werden kann) und wird abgetrennt. Die verbleibende organische Phase enthält neben Anilin noch organische Verunreinigungen und gelöstes Wasser.

In vorteilhafter Weise lässt sich die Hydrierung in der Gasphase durchführen, wobei Wasserstoff bevorzugt im stöchiometrischen Überschuss eingesetzt wird. Dabei kann die Hydrierung, wie dem Fachmann bekannt ist, adiabatisch (ohne gezielte Abfuhr der Reaktionswärme) oder isotherm (unter gezielter Abfuhr der Reaktionswärme, bspw. in einem Rohrbündelreaktor unter Einsatz eines kühlenden Wärmeträgermediums wie Wasser, eines Öls oder einer Salzschmelze) durchgeführt werden.

Bei adiabatischer Fahrweise werden in der Regel mehrere Reaktoren in Serie hintereinander angeordnet. Hier führt die Reaktionswärme zu einer beträchtlichen Temperaturerhöhung des Reaktionsgases (sog. "adiabatischer Temperatursprung"), sodass dieses nach Austritt aus einem Reaktor gekühlt wird. Nach Abkühlung werden optional erneut gasförmiges Nitrobenzol und weiterer Wasserstoff hinzugegeben und einem weiteren Reaktor zugeführt. Zur Verdünnung der Reaktionswärme wird in adiabatischen Verfahren im Allgemeinen mit besonders großen Wasserstoffüberschüssen gearbeitet. Nicht verbrauchter Wasserstoff wird daher bevorzugt im Kreis gefahren.

Beim isothermen Verfahren wird in der Regel ein einziger Reaktor verwendet. Dieser wird wie beschrieben aktiv gekühlt. Trotz der im Vergleich zum adiabatischen Verfahren in der Regel deutlich kleineren Wasserstoffüberschüsse ist eine Kreisführung nicht verbrauchten Wasserstoffs meist sinnvoll.

Die Vorteile des adiabatisch durchgeführten Verfahrens liegen in der einfacheren Bauweise der Reaktoren, da keine Kühlung des Katalysatorbettes notwendig ist. Das isotherme Verfahren ist durch die notwendige Kühlung des Katalysatorbettes entsprechend aufwändiger. Aufgrund der realisierbaren Reaktorgrößen ist hier jedoch im Allgemeinen keine serielle Anordnung mehrerer Reaktoren und der entsprechenden Zwischenkühler notwendig.

Das erfindungsgemäße Verfahren ist insbesondere dann vorteilhaft einsetzbar, wenn die Hydrierung isotherm unter Einsatz eines Palladium auf einem Aluminiumoxidträger enthaltenden Katalysators oder adiabatisch unter Einsatz eines Kupfer auf einem Siliciumdioxidträger enthaltenden Katalysators durchgeführt wird. In beiden Fällen entstehen neben Phenol verhältnismäßig große Mengen an Aminophenolen, die durch das erfindungsgemäße Verfahren auf effiziente Weise abgetrennt werden können.

Das in beiden Verfahrensweisen zunächst erhaltene gasförmige Reaktionsprodukt wird kondensiert. Eine solche Kondensation kann einstufig (d. h. unter Erhalt eines einzigen Kondensats) erfolgen, woran sich eine Phasentrennung anschließt. Die nach Abtrennung des Reaktionswassers verbleibende organische Phase kann dem Schritt b) als *Rohanilinfraktion* zugeführt werden.

Es ist jedoch auch möglich, die Kondensation zweistufig derart durchzuführen, dass zwei Kondensate *unterschiedlicher Zusammensetzung* erhalten werden. Hierzu wird der zunächst anfallende gasförmige Rohproduktstrom sukzessive fallenden Temperaturen ausgesetzt, sodass zunächst nur die höher siedenden Komponenten zusammen mit Anilin auskondensieren. Auf diese Weise wird ein erstes Kondensat (das sog. "Partialkondensat") erhalten, das kaum Wasser enthält und bevorzugt einphasig ist, sodass eine Phasentrennung hier überflüssig ist. Diese "Partialkondensat" besteht also im Wesentlichen aus Anilin und Hochsiedern. Erst in der bei tieferer Temperatur durchgeführten zweiten Kondensation (der sog. "Totalkondensation") kondensieren neben weiterem Anilin auch Leichtsieder und Wasser aus. Die Phasentrennung zur Abtrennung des Reaktionswassers kann daher auf dieses "Totalkondensat" beschränkt bleiben. Die nach der Phasentrennung verbleibende organische Phase kann vorteilhaferweise mit dem Partialkondensat vereinigt und als Rohanilinfraktion dem Schritt b) zugeführt werden. Es ist jedoch auch möglich, nur einen der beiden Ströme - Partialkondensat oder die nach Phasentrennung erhaltene organische Phase des Totalkondensats - als Rohanilinfraktion dem Schritt b) zuzuführen.

Unabhängig von der genauen Art der Bereitstellung des Rohanilins ist die vorliegende Erfindung für die Reinigung von Rohanilinfraktionen mit einer auf die Gesamtmasse der organischen Bestandteile der Rohanilinfraktion bezogenen Konzentration an Aminophenol im Bereich von 0,001 Massen-% bis 1,00 Massen-%, bevorzugt im Bereich von 0,001 Massen-% bis 0,50 Massen-%, besonders bevorzugt im Bereich von 0,001 Massen-% bis 0,10 Massen-%, anwendbar. Da die Bildung von Aminophenol stets mit der Bildung von Phenol einhergeht, enthalten solche Rohanilinfraktionen regelmäßig auch Phenol, und zwar insbesondere im Bereich von 0,001 Massen-% bis 1,00 Massen-%, bevorzugt im Bereich von 0,001 Massen-% bis 0,50 Massen-%, besonders bevorzugt im Bereich von 0,001 Massen-% bis 0,10 Massen-%, bezogen auf die Gesamtmasse der organischen Bestandteile der Rohanilinfraktion.

Solange das erfindungsgemäße Erfordernis des zusätzlichen Einsatzes eines *von einem Alkalimetallhydroxid verschiedenen Alkalimetallsalzes* beachtet wird, kann die Extraktion in Schritt b) grundsätzlich mit im Stand der Technik an sich bekannten Apparaten und Vorgehensweisen durchgeführt werden. Ohne auf eine Theorie festgelegt werden zu wollen wird angenommen, dass das von einem Alkalimetallhydroxid verschiedene Alkalimetallsalz auch bei vergleichsweise geringen Alkalimetallhydroxid-konzentrationen im Extraktionsmittel einen ausreichenden Dichteunterschied zwischen wässriger und organischer Phase gewährleistet und so eine einfache Phasentrennung begünstigt. Eine solche Begünstigung der Trennung zweier nicht oder nur sehr wenig mischbarer Phasen ist zu unterscheiden von der in organischen Chemie bekannten Vorgehensweise, die *Löslichkeit* organischer Verbindungen in einer wässrigen Phase durch Sättigung der Letzteren mit Salz herabzusetzen und so in der Aufarbeitung wässrig-organischer Verfahrensprodukte die Ausbeute an organischem Zielprodukt zu erhöhen (indem der Verlust über dessen Restlöslichkeit in der wässrige Phasen minimiert wird).

Die beiden erfindungswesentlichen Bestandteile des wässrigen Extraktionsmittels, das Alkalimetallhydroxid (der eigentliche reaktive Bestandteil) und das von einem Alkalimetallhydroxid verschiedene Alkalimetallsalz, können der Extraktion in vorvermischter Form (was bevorzugt ist) oder auch getrennt (über getrennte Zuführeinrichtungen) zugeführt werden. Die Extraktion kann ein- oder mehrstufig in dem Fachmann bekannten Apparaten, insbesondere in sog. "Mixer-Settler"-Apparaturen (Mischabsetzer), durchgeführt werden. Besonders bevorzugt ist eine mehrstufige, insbesondere zweistufige, Extraktion im Gegenstrom. Die Phasen werden nach jedem Extraktionsschritt getrennt, und die nach dem letzten (gegebenenfalls einzigen) Extraktionsschritt erhaltene organische Phase, die an Aminophenol (und natürlich auch an Phenol) abgereichert ist, wird dem Schritt c) zugeführt.

Bevorzugt wird die Extraktion in Schritt b) bei einer Temperatur im Bereich von 20 °C bis 95 °C, besonders bevorzugt 25 °C bis 85 °C, ganz besonders bevorzugt 25 °C bis 70 °C und außerordentlich ganz besonders bevorzugt 25 °C bis 40 °C durchgeführt.

Erfindungsgemäß werden, bezogen auf die Gesamtmasse des Extraktionsmittels, das Alkalimetallhydroxid in einem Konzentrationsbereich von 0,009 Massen-% bis 2,05 Massen-%, bevorzugt 0,010 Massen-% bis 2,00 Massen-%, besonders bevorzugt im Konzentrationsbereich von 0,010 Massen-% bis 1,00 Massen-%, ganz besonders bevorzugt im Konzentrationsbereich von 0,50 Massen-% bis 1,00 Massen-%, außerordentlich ganz besonders bevorzugt im Konzentrationsbereich von 0,76 Massen-% bis 1,00 Massen-%, und das von einem Alkalimetallhydroxid verschiedene Alkalimetallsalz in einem Konzentrationsbereich von 2,40 Massen-% bis 25,0 Massen-%, bevorzugt 4,00 Massen-% bis 25,0 Massen-%, besonders bevorzugt im Konzentrationsbereich von 5,00 Massen-% bis 25,0 Massen-%, ganz besonders bevorzugt im Konzentrationsbereich von 10,0 Massen-% bis 20,0 Massen-%, außerordentlich ganz besonders bevorzugt im Konzentrationsbereich von 12,0 Massen-% bis 17,0 Massen-%, eingesetzt. Die Vorvermischung der beiden erfindungswesentlichen Bestandteile ist wie bereits erwähnt bevorzugt. Bei der ebenfalls möglichen getrennten Zufuhr der beiden erfindungswesentlichen Bestandteile in den für Schritt b) verwendeten Extraktionsapparat (bei der sich das Extraktionsmittel erst im Extraktionsapparat durch Vermischung bildet) ist die Bezugsgröße für die genannten Massenkonzentrationen die Summe der Einzelmassen der beiden erfindungswesentlichen Bestandteile (die gleich der Masse des vorvermischten Extraktionsmittels in der bevorzugten Ausführungsform ist).

In einer bevorzugten Ausführungsform wird die Gesamtkonzentration an Phenol und Aminophenolen in der Rohanilinfraktion kontinuierlich oder in Intervallen überwacht und die Zusammensetzung und/oder die Menge des Extraktionsmittels darauf abgestimmt. Für eine möglichst vollständige Abtrennung von Aminophenol (und Phenol) muss das Alkalimetallhydroxid in, bezogen auf phenolische Hydroxygruppen, mindestens stöchiometrischer Menge, vorzugsweise im Überschuss, eingesetzt werden. (Als *phenolische Hydroxygruppen* werden im Rahmen der vorliegenden Erfindung die Hydroxygruppen von Phenol und Aminophenolen summarisch bezeichnet; Phenol und Aminophenole werden auch summarisch als *phenolische Verbindungen* bezeichnet.) Unter Umständen kann es jedoch auch sinnvoll sein, in Schritt b) bewusst nicht alle phenolischen Verbindungen zu neutralisieren und stattdessen einen Teil der Abtrennung in die sich anschließende Destillation von Schritt c) zu verlagern. Wenn die Destillation in Schritt c) eine bestimmte Menge an phenolischen Verbindungen problemlos abtrennen kann, kann auf diese Weise der Einsatz des Extraktionsmittels in Schritt b) minimiert werden. Abhängig von der genauen Vorgehensweise kann daher die vorteilhafterweise einzusetzende relative Menge an Alkalimetallhydroxid variieren. Insbesondere haben sich molare Verhältnisse von Alkalimetallhydroxid zu phenolischen Hydroxygruppen im Bereich von 0,80 bis 50, bevorzugt im Bereich von 0,95 bis 10, besonders bevorzugt im Bereich von 1,0 bis 5,0 und ganz besonders bevorzugt im Bereich von 1,0 bis 2,4, bewährt. Die molaren Verhältnisse können im Rahmen der vorliegenden Erfindung allein an dem Ziel ausgerichtet werden, die Konzentration an phenolischen Hydroxygruppen unter den vorliegenden Bedingungen soweit wie erforderlich zu verringern. Es ist insbesondere nicht erforderlich, darüber hinaus weiteres Alkalimetallhydroxid zuzugeben, um eine gewünschte Dichte einzustellen, wie dies bei den Verfahren des Standes der Technik durchaus erforderlich sein kann. Dagegen können eventuell erforderliche Einstellungen der Dichte im erfindungsgemäßen Verfahren auf einfache und kostengünstige Weise durch die Zugabe des von einem Alkalimetallhydroxid verschiedenen Alkalimetallsalzes vorgenommen werden.

Es kann auch sinnvoll sein, die in der Extraktion erhaltene an Aminophenol abgereicherte Anilinphase vor deren Destillation in Schritt c) mit weiterem wässrigem Alkalimetallhydroxid zu versetzen und die so erhaltene Mischung ohne Abtrennung ihrer wässrigen Bestandteile der Destillation von Schritt c) zu unterwerfen. Dies kommt einer Kombination aus der erfindungsgemäßen basischen Extraktion in Gegenwart eines von einem Alkalimetallhydroxid verschiedenen Alkalimetallsalzes und der literaturbekannten Destillation in Gegenwart von Base gleich.

Als Alkalimetallhydroxid wird vorzugsweise Natrium- oder Kaliumhydroxid eingesetzt. Als ein von einem Alkalimetallhydroxid verschiedenes Alkalimetallsalz wird vorzugsweise Natrium- bzw. Kaliumchlorid oder Natrium- oder Kaliumsulfat eingesetzt. Das Alkalimetallsalz des verwendeten Hydroxids sollte vorzugsweise das Gleiche sein wie das des verwendeten von einem Alkalimetallhydroxid verschiedenen Alkalimetallsalzes, insbesondere jeweils Natrium oder Kalium.

In Schritt c) erfolgt die destillative (Fein-)Reinigung des durch die Extraktion in Schritt b) vorgereinigten Rohanilins. Grundsätzlich sind alle dem Fachmann als für die Anilinreinigung geeignet bekannten Destillationsvorrichtungen und -verfahren einsetzbar.

In einer ersten Ausgestaltungsmöglichkeit des Schritts c) der vorliegenden Erfindung erfolgt die Abtrennung der Leichtsieder und gelösten Wassers einerseits und der Hochsieder andererseits in getrennten Destillationskolonnen, d. h. die destillative Reinigung umfasst zwei hintereinandergeschaltete Destillationskolonnen. Der Zulauf der ersten Destillationskolonne ist die durch basische Extraktion vorgereinigte Rohanilinfraktion aus Schritt b).

In der ersten Destillationskolonne (der sog. "Leichtsiederkolonne") werden Leichtsieder über Kopf abdestilliert, während Anilin und Hochsieder im Sumpf anfallen. Gelöstes Wasser kann ebenfalls über Kopf oder aber auch in einem Seitenabzug abgezogen werden. Der Sumpf der ersten Kolonne bildet den Zulauf einer weiteren (der zweiten) Destillationskolonne (der sog. "Anilinkolonne"), in welcher Anilin über Kopf abdestilliert wird und in deren Sumpf ein Anilin-haltiger Hochsiederstrom anfällt. Das über Kopf abdestillierte Anilin stellt in dieser Ausführungsform den Strom gereinigten Anilins im Sinne der Erfindung dar.

In der ersten Ausgestaltungsmöglichkeit kann die "Leichtsiederdestillation" vorzugsweise bei einem Kopfdruck im Bereich von 1000 mbar_{(abs.)} bis 1100 mbar_{(abs.)} und einer Kopftemperatur im Bereich von 97 °C bis 100 °C durchgeführt werden. Die Destillation in der Anilinkolonne wird vorzugsweise bei einem Kopfdruck im Bereich von 100 mbar_{(abs.)} bis 200 mbar_{(abs.)} und einer Kopftemperatur im Bereich von 110 °C bis 130 °C durchgeführt.

In einer zweiten Ausgestaltungsmöglichkeit des Schrittes c) der vorliegenden Erfindung erfolgt die Abtrennung gelösten Wassers in einer eigenen Destillationskolonne (der sog. "Entwässerungskolonne"). Die durch basische Extraktion vorgereinigte Rohanilinfraktion aus Schritt b) wird dieser Entwässerungskolonne (in dieser Ausgestaltungsmöglichkeit die erste Destillationskolonne) zugeführt. Der Entwässerungskolonne wird ein Wasser-, Anilin- und Leichtsieder-haltiger Strom am Kopf entnommen, welcher anschließend in eine wässrige und organische Phase getrennt wird. Die organische Phase wird in eine weitere Destillationskolonne (die "Leichtsiederkolonne", in dieser Ausgestaltungsmöglichkeit die zweite Destillationskolonne) geführt, in welcher Leichtsieder über Kopf abdestilliert werden. Der Sumpfstrom dieser Leichtsiederkolonne wird in die Entwässerungskolonne zurückgeführt.

Der Sumpf der Entwässerungskolonne bildet den Zulauf einer dritten Destillationskolonne (der sog. "Anilinkolonne"), in welcher wie in der ersten Ausgestaltungsmöglichkeit Anilin über Kopf abdestilliert wird und in deren Sumpf ein Anilin-haltiger Hochsiederstrom anfällt. Das über Kopf abdestillierte Anilin stellt wie in der ersten Ausgestaltungsmöglichkeit den Strom gereinigten Anilins im Sinne der Erfindung dar.

In der zweiten Ausgestaltungsmöglichkeit kann die "Entwässerungskolonne vorzugsweise bei einem Kopfdruck im Bereich 100 mbar_{(abs.)} bis 400 mbar_{(abs.)} und eine Kopftemperatur im Bereich von 100 °C bis 150 °C betrieben werden. Die "Leichtsiederdestillation wird bevorzugt bei einem Kopfdruck im Bereich von 1000 mbar_{(abs.)} bis 1100 mbar_{(abs.)} und einer Kopftemperatur im Bereich von 70 °C bis 90 °C durchgeführt. Die Destillation in der Anilinkolonne wird vorzugsweise bei einem Kopfdruck im Bereich von 250 mbar_{(abs.)} bis 350 mbar_{(abs.)} und einer Kopftemperatur im Bereich von 140 °C bis 160 °C durchgeführt.

Es ist jedoch auch möglich und Gegenstand einer dritten Ausgestaltungsmöglichkeit des Schritts c) der vorliegenden Erfindung, die durch basische Extraktion vorgereinigte Rohanilinfraktion aus Schritt b) in eine Destillationskolonne mit Seitenabzug (auch als Seitenabzugskolonne bezeichnet) einzuführen, in der Leicht- und Hochsieder (sowie gelöstes Wasser) *in einem einzigen Destillationsschritt* abgetrennt werden. In einer Ausführungsform der dritten Ausgestaltungsmöglichkeit verfügt die Seitenabzugskolonne über eine Trennwand (sog. Trennwandkolonne). In der dritten Ausgestaltungsmöglichkeit wird der Strom gereinigten Anilins der Destillationskolonne als Seitenabzug entnommen, während Leichtsieder (und Wasser) über Kopf abgezogen werden. Im Sumpf fällt, wie in der Anilinkolonne der zuvor genannten Ausgestaltungsmöglichkeiten, ein Anilin-haltiger Hochsiederstrom an. Diese Destillation wird vorzugsweise bei einem Kopfdruck der Seitenabzugskolonne im Bereich von 200 mbar_{(abs.)} bis 500 mbar_{(abs.)} und bei einer Kopftemperatur der Seitenabzugskolonne im Bereich von 100 °C bis 150 °C durchgeführt.

In allen Fällen fällt der Leichtsiederstrom (also in der ersten Ausgestaltungsmöglichkeit der Kopfstrom der ersten Destillationskolonnne, in der zweiten Ausgestaltungsmöglichkeit der Kopfstrom der zweiten Destillationskolonne und in der dritten Ausgestaltungsmöglichkeit der Kopfstrom der Seitenabzugskolonnne) zunächst gasförmig an. Dieser gasförmige Leichtsiederstrom wird bevorzugt partiell verflüssigt, wobei die so erhaltene flüssige Phase, optional nach Abtrennung gegebenenfalls vorhandenen Wassers, in vorteilhafterweise als Rücklauf wieder auf die Ausgangskolonne gegeben werden kann. Die nicht kondensierte Gasphase, die neben einem Anteil an (unter industrieüblichen Bedingungen) nicht kondensierbaren Gasen wie beispielsweise Stickstoff im Wesentlichen aus Leichtsiedern besteht, wird aus der Destillation ausgetragen.

Es kann vorteilhaft sein, den in den genannten Ausgestaltungsmöglichkeiten für Schritt c) anfallenden Anilin-haltigen Hochsiederstrom in einem Schritt d.1) einer Aufkonzentrierung der Hochsieder zu unterwerfen, um auf diese Weise den Verlust an Wertprodukt Anilin zu minimieren (entsprechend der weiter oben erwähnten ersten Variante). Dabei wird ein Anilin-haltiger Strom verdampft, der vorteilhafterweise in die Destillation aus Schritt c) zurückgeführt werden kann. Bevorzugt wird dieser Anilin-haltige Strom in den Sumpf derjenigen Destillationskolonne geführt, aus welcher der Anilin-haltige Hochsiederstrom ursprünglich stammte. Daneben fällt in diesem Schritt ein höher als Anilin siedende organische Verunreinigungen enthaltender Sumpfstrom (Hochsiederstrom) an. Die Aufkonzentrierung gemäß Schritt d.1) erfolgt bevorzugt in einer eigens dafür vorgesehenen Destillationskolonne.

Die Wirtschaftlichkeit des erfindungsgemäßen Verfahrens kann in der ersten Variante weiter verbessert werden, wenn die im Extraktionsschritt Schritt b) erhaltene wässrige Aminophenolatphase zur Überführung von Aminophenolat in Aminophenol in einem Schritt e.1) mit Säure behandelt und in einem Schritt f.1) mit einem organischen Strom extrahiert wird, wobei nach Phasentrennung eine organische (an Aminophenol angereicherte) Phase und eine wässrige (an Aminophenol abgereicherte) Phase erhalten werden. Es ist bevorzugt, den Schritt f.1) parallel zum Schritt e.1) durchzuführen (= simultane Zugabe von Säure und organischem Strom zur wässrigen Aminophenolatphase), um zu verhindern, dass Aminophenole als Feststoffe ausfallen. Diese wässrige Phase kann dann vorteilhafterweise mit Alkalimetallhydroxid versetzt und als Bestandteil des in Schritt a) einzusetzenden Extraktionsmittels verwendet werden. Es ist möglich, den gesamten Anteil des in Schritt a) einzusetzenden Alkalimetallhydroxids auf diese Weise zuzuführen. Es ist ebenfalls möglich, den gesamten Anteil des in Schritt a) einzusetzenden von einem Alkalimetallhydroxid verschiedenen Alkalimetallsalzes auf diese Weise, d. h. in Form des in dieser Ausführungsform ohnehin in der wässrigen (an Aminophenol abgereicherten) Phase vorhandenen Salzes *der in Schritt e.1) eingesetzten Säure* zuzuführen. Als organischer Strom zur Extraktion eignet sich besonders der in Schritt d.1) anfallende Hochsieder-Sumpfstrom. Dieser Hochsieder-Sumpfstrom kann zu diesem Zweck auch mit anderen organischen Strömen gemischt werden. So kann etwa die in Schritt c) bei der bevorzugt durchgeführten partiellen Verflüssigung des gasförmigen Leichtsieder-Kopfstroms anfallende nicht kondensierte Gasphase, die neben einem Anteil an (unter industrieüblichen Bedingungen) nicht kondensierbaren Gasen wie beispielsweise Stickstoff im Wesentlichen aus Leichtsiedern besteht, durch Verflüssigung ihrer kondensierbaren Anteile in einen flüssigen Leichtsiederstrom überführt werden, der sich zur Abmischung mit der in Schritt d.1) anfallenden Hochsieder-Sumpfstrom zum Zwecke der Extraktion von Aminophenol eignet. In jedem Fall ist der Anilingehalt der nach Extraktion und Phasentrennung erhaltenen Aminophenol-haltigen organischen Phase so gering, dass diese organische Phase ohne nennenswerte wirtschaftliche Verluste verbrannt werden kann. In Schritt f.1) werden Aminophenole (und soweit vorhanden selbstverständlich auch Phenole) wieder von einer wässrigen Phase in eine organische Phase zurück überführt (sie stammen ja ursprünglich aus einer organischen Phase, der Rohanilinfraktion); daher wir dieser Schritt auch als Rückextraktion bezeichnet.

Auch die weiter oben beschriebene zweite Variante macht von einer Rückextraktion in diesem Sinne Gebrauch (hier Schritt e.2)). Als organischer Strom dient hier jedoch im Unterschied zu ersten Variante der *höher als Anilin siedende organische Verunreinigungen und Anilin enthaltende flüssigen Strom aus Schritt c),* während die Säurebehandlung (hier Schritt d.2)) grundsätzlich wie in der ersten Variante durchgeführt werden kann. Im Unterschied zur ersten Variante wird hier *die in der Rückextraktion anfallende organische Phase* aufkonzentriert (in Schritt f.2)), wobei dieser Schritt so auszugestalten ist, dass ein Wiedereinführen von Aminophenolen/Phenolen über das Destillat von Schritt f.2) in Schritt c) vermieden wird. Aufgrund der vergleichsweise geringen Größe des Stroms an organischer Phase, welcher der Aufkonzentrierung zugeführt wird, ist dies jedoch möglich, ohne die energetischen Vorteile des Verfahrens zu gefährden. Der Vorteil dieser zweiten Variante ist in einer robusteren Prozessführung zu sehen, die größere Freiheitsgrade bietet. Die Menge des organischen Stroms, welcher der Rückextraktion zugeführt wird - hier Strom 9 -, kann in der zweiten Variante erforderlichenfalls größer gewählt werden, als in der ersten Variante (dort ist die Menge dieses organischen Stroms - Strom 18 - durch die Konzentration an Hochsiedern in der Rohanilinfraktion im Wesentlichen festgelegt). In Strom 9 enthaltenes Anilin gelangt in Strom 19 und wird in die Destillationskolonne zur Aufkonzentrierung der Hochsieder (5000) zurückgeführt, von wo es über Strom 17 in die Anilindestillation (3000) gelangt, also nicht verloren geht. In der zweiten Variante kann daher die Menge des organischen Stroms - hier der höher als Anilin siedende organische Verunreinigungen und Anilin enthaltende flüssige Strom aus Schritt c) (Strom 9) - unabhängig von der Menge an anfallenden Hochsiedern gewählt werden. So ist eine Phasentrennung in einem für industrielle Anwendungen relevanten Maßstab in e.2) für sämtliche Zusammensetzungen der Rohanilinfraktion durchführbar. Auch in der zweiten Variante ist es bevorzugt, den Schritt der Extraktion mit dem organischen Strom (hier Schritt e.2) parallel zum Schritt der Säurebehandlung (hier Schritt d.2)) durchzuführen (= simultane Zugabe von Säure und organischem Strom zur wässrigen Aminophenolatphase), um zu verhindern, dass Aminophenole als Feststoffe ausfallen.

Als Säure für die Säurebehandlung gemäß Schritt e.1) bzw. Schritt d.2) eignen sich insbesondere Salzsäure oder Schwefelsäure. Die Wahl der Säure erfolgt entsprechend der Wahl des in Schritt a) eingesetzten von einem Alkalimetallhydroxid verschiedenen Alkalimetallsalz; im Fall eines Alkalimetallchlorids (insbesondere Natrium- oder Kaliumchlorid) wird Salzsäure, im Falle eines Alkalimetallsufats (insbesondere Natrium- oder Kaliumsulfat) wird Schwefelsäure eingesetzt. Die Säurebehandlung in Schritt e.1) bzw. Schritt d.2) erfolgt vorzugsweise bis zu einem pH-Wert (20 °C) im Bereich von 2,0 bis 8,5. bevorzugt bis zu einem pH-Wert (20 °C) im Bereich von 3,5 bis 8,5, besonders bevorzugt bis zu einem pH-Wert (20 °C) im Bereich von 5,0 bis 8,5.

FIG. 2a zeigt eine bevorzugte Ausgestaltung des erfindungsgemäßen Verfahrens in der ersten Variante mit zweistufiger Kondensation des Rohprodukts einer Gasphasen-Anilinhydrierung; Aufkonzentration des Sumpfstromes der Anilindestillation und Extraktion des in der Basenextraktion erhaltenen Abwassers mit dem Hochsiederstrom aus dem Aufkonzentrierungschritt. FIG. 2b zeigt eine entsprechende Ausgestaltung für die zweite Variante. Identische Bezugszeichen wie in FIG. 1 haben die jeweils gleiche Bedeutung wie dort. Die weiteren Bezugszeichen haben die folgende Bedeutung.

### Apparate bzw. Verfahrensschritte:

| | |
|---|---|
| 4100 | Partialkondensation; |
| 4200 | Mischer; |
| 4300 | Totalkondensation; |
| 4400 | Phasentrennung; |
| 5000 | Destillationskolonne zur Aufkonzentrierung der Hochsieder; |
| 6000 | Neutralisation; |
| 7000 | Extraktion inklusive Phasentrennung. |

### Stoffströme:

| | |
|---|---|
| 11 | Gasförmiges Rohprodukt der Anilin-Hydrierung; |
| 12 | Partialkondensat; |
| 13 | in der Partialkondensation nicht kondensierte Anteile des Rohprodukts; |
| 14 | Totalkondensat; |
| 15 | in der Totalkondensation nicht kondensierte Anteile (im Wesentlichen überschüssiger Wasserstoff); |
| 16 | organische Phase des Totalkondensats; |
| 17 | Anilin-haltiger Destillatstrom; |
| 18 | Hochsieder enthaltender Sumpfstrom (in der zweiten Variante gemäß FIG. 2b: bevorzugt zur Verbrennung); |
| 19 | Hochsiederstrom aus Extraktion (in der ersten Variante gemäß FIG. 2a: bevorzugt zur Verbrennung); |
| 20 | wässrige (an Aminophenol abgereicherte) Phase (wird Strom 3 zugemischt; ein Teil kann auch als Abwasser ausgetragen werden) |
| 21 | Säure (bevorzugt Salz- oder Schwefelsäure). |

Die Darstellung der Neutralisation und Extraktion (inklusive der Phasentrennung) in FIG. 2a/b in zwei Stufen 6000 und 7000 bedeutet nicht, dass diese Verfahrensschritte notwendigerweise in zwei Apparaturen durchgeführt werden müssen. In einer bevorzugten Ausführungsform wird die Extraktion wie bereits erwähnt in einer sog. "Mixer-Settler"-Apparatur durchgeführt, d. h. einer Apparatur, die eine Mischeinheit und eine Trenneinheit umfasst. In einer besonders bevorzugten Ausgestaltung dieser Ausführungsform werden die Säure (Strom 21 in den Abbildungen) und der organische Strom (Strom 18 bzw. Strom 9 in den Abbildungen FIG. 2a bzw. FIG. 2b) in die Mischeinheit eingeführt, gefolgt von einer Phasentrennung in der Trenneinheit.

### Beispiele:

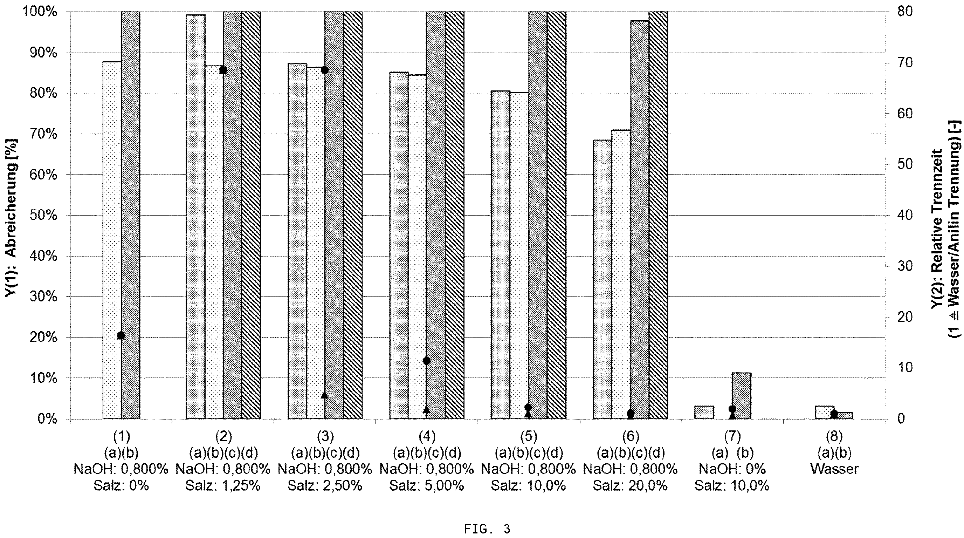

### Beispiel 1: Prinzipversuche zur Abreicherung von Aminophenol und zur Phasentrennzeit (siehe FIG. 3)

Das zu reinigende Rohanilin enthielt, bezogen auf die Gesamtmasse seiner organischen Bestandteile, 750 ppm Phenol und 500 ppm Aminophenole und war mit Wasser gesättigt. Dieses Gemisch wurde in verschiedenen Versuchen mit Wasser, Natronlauge, Natriumchloridlösung und Lösungen enthaltend Natriumhydroxid und Natriumchlorid extrahiert, wobei ein Volumenverhältnis von Anilinphase zu Extraktionsmittel von 4 : 1 eingehalten wurde. Dabei wurde die NaOH-Konzentration konstant auf einen Wert von 0,800 Massen-% bei verschiedenen NaCl-Konzentrationen (0 Massen-%, 1,25 Massen-%, 2,50 Massen-%, 5,00 Massen-%, 10,0 Massen-% und 20,0 Massen-%) eingestellt. Die Versuchsreihe wurde in analoger Weise auch mit Natriumsulfat-Lösungen anstelle von NaCl-Lösungen durchgeführt.

Es zeigte sich, dass im Versuch mit einer Salzkonzentration von 5,00 Massen-% die Phasentrennung im Fall von beiden Salzen, NaCl und Na₂S0₄, erheblich beschleunigt wurde. Im Versuch mit einer Salzkonzentration von 10,0 Massen-% war die zur Phasentrennung benötigte Zeit im Fall von beiden Salzen vergleichbar mit der des reinen Wasser-Anilin-Gemisches. Bei einer Salzkonzentration von 2,50 Massen-% wurde im Fall von Natriumsulfat ebenfalls eine erhebliche Verkürzung der Phasentrennzeit beobachtet.

Bei allen Versuchen (außer bei Verwendung reinen Wassers und reiner Natriumchloridlösung) wurde das ortho-Aminophenol bis unter die Nachweisgrenze aus der organischen Phase entfernt, das zugesetzte Phenol konnte dabei zu 75 % bis 85 % aus dem Rohanilin entfernt werden. Bei allen Versuchen wurde keine Feststoffbildung beobachtet.

Die Ergebnisse sind in FIG. 3 in Form eines Diagramms dargestellt.

Auf der linken Ordinatenachse (Y1) ist die prozentuale Abreicherung von Phenol bzw. ortho-Aminophenol dargestellt, mit Bezug auf die Höhe der Säulen (beispielsweise bedeutet ein Wert von 100 auf der linken Ordinatenachse, dass die entsprechende phenolische Verbindung bis unter die Nachweisgrenze abgereichert wurde).

Auf der rechten Ordinatenachse (Y2) ist die relative Trennzeit (Zeitbedarf für die Phasentrennung) dargestellt, mit Bezug auf die Lage der Dreiecke (Trennzeit für Versuche mit Natriumsulfat bzw. der Kreise (Versuche mit Natriumchlorid und Versuche ohne die Zugabe eines weiteren Salzes). Zu diesem Zweck wurde der Zeitbedarf für die Trennung von Anilin der oben genannten Zusammensetzung von vollentsalztem Wasser zu 1 (= Referenz) angesetzt, und die übrigen Werte wurden in Relation dazu angegeben (beispielsweise bedeutet ein Wert von 80 auf der rechten Ordinatenachse, dass die Phasentrennung 80-mal so lange dauert wie im Referenzfall).

Auf der Abszissenachse ist das Ergebnis einer jeden Versuchsreihe in Form mehrerer Säulen bzw. Symbole (Dreiecke, Kreise) dargestellt, welche die zuvor genannte Bedeutung haben. Von links nach rechts sind dabei die nachfolgenden Versuchsreihen gezeigt.

### Extraktion des Rohanilins mit :

(1) Natronlauge ohne weiteren Salzgehalt,
(2) Natronlauge enthaltend einen Massenanteil an weiterem Salz von 1,25 %,
(3) Natronlauge enthaltend einen Massenanteil an weiterem Salz von 2,50 %,
(4) Natronlauge enthaltend einen Massenanteil an weiterem Salz von 5,00 %,
(5) Natronlauge enthaltend einen Massenanteil an weiterem Salz von 10,0 %,
(6) Natronlauge enthaltend einen Massenanteil an weiterem Salz von 20,0 %,
(7) Wasser (ohne NaOH) enthaltend einen Massenanteil an Salz von 10,0 % bzw.
(8) vollentsalztem Wasser (weder NaOH noch weiteres Salz enthaltend).

In Versuchsreihe (1) bezeichnen die Säulen von links nach rechts die prozentuale Abreicherung von :
(a) Phenol bzw.
(b) ortho-Aminophenol,
jeweils ohne die Zugabe eines weiteren Salzes.

In den Versuchsreihen (2) bis (6) bezeichnen die Säulen von links nach rechts die prozentuale Abreicherung von :
(a) Phenol bei Verwendung von Natriumsulfat als weiterem Salz,
(b) Phenol bei Verwendung von Natriumchlorid als weiterem Salz,
(c) ortho-Aminophenol bei Verwendung von Natriumsulfat als weiterem Salz bzw.
(d) ortho-Aminophenol bei Verwendung von Natriumchlorid als weiterem Salz.

In Versuchsreihe (7) bezeichnen die Säulen von links nach rechts die prozentuale Abreicherung von :
(a) Phenol bei Verwendung von Natriumsulfat als weiterem Salz bzw.
(b) ortho-Aminophenol bei Verwendung von Natriumsulfat als weiterem Salz.

In Versuchsreihe (8) bezeichnen die Säulen von links nach rechts die prozentuale Abreicherung von :
(a) Phenol bzw.
(b) ortho-Aminophenol,
jeweils ohne die Zugabe eines weiteren Salzes.

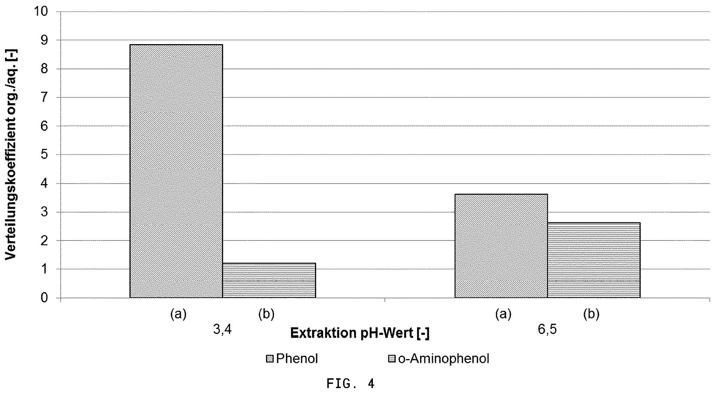

### Beispiel 2: Prinzipversuche zur Neutralisation der an Aminophenol abgereicherten wässrigen Phase und anschließender Extraktion des gebildeten Aminophenols (siehe FIG. 4)

Eine wässrige NaOH-Lösung (Konzentration 0,800 Massen-%) wurde mit Anilin gesättigt und anschließend mit 5000 ppm ortho-Aminophenol und 5000 ppm Phenol dotiert, so dass die Salze der der jeweiligen phenolischen Verbindungen vorlagen. Unterschiedliche Probe(n) wurden daraufhin mit Salzsäure einer Konzentration von 30 Massen-% auf einen pH-Wert im Bereich von 6,0 bis 7,0 und unter 3,5 eingestellt. Die angesäuerten Proben wurden nun mit einem synthetischen Hochsiederabfallstrom, bestehend aus Diphenylamin (als exemplarischer Hochsieder) und Anilin im Massenverhältnis 1 : 1 extrahiert. Dabei war es möglich, die Konzentration aller phenolischen Verbindungen in dem wässrigen Medium um den Faktor 1,5 bis 9,0, je nach pH-Wert und Art der phenolischen Verbindung, zu reduzieren. Man erkennt zudem, dass sich die phenolischen Verbindungen abhängig vom pH-Wert unterschiedlich gut extrahieren lassen.

Die Ergebnisse sind in FIG. 4 in Form eines Säulendiagramms dargestellt.

Auf der Ordinatenachse (Y) ist der Verteilungskoeffizient der jeweiligen phenolischen Verbindung als Verhältnis von deren Massenanteil in der organischen Phase zu deren Massenanteil in der wässrigen Phase mit Bezug auf die Höhe der Säulen angegeben.

Auf der Abszissenachse (x) ist das Ergebnis einer jeden Versuchsreihe in Form von jeweils zwei Säulen bei den pH-Werten 3,4 und 6,5 gezeigt. Dabei zeigt die jeweils linke Säule (a) den Verteilungskoeffizienten von Phenol und die jeweils rechte Säule (b) den Verteilungskoeffizienten von ortho-Aminophenol an.

### Beispiel 3: Kontinuierliche Versuche zum Verfahren

Betriebsproben eines Rohanilins (enthaltend ca. 2500 ppm Phenol und 250 ppm Aminophenol) wurden kontinuierlich in einer zweistufigen Mixer-Settler-Anlage mit einer wässrigen Lösung enthaltend 10 Massen_% Na₂S0₄ und 1 Massen-% NaOH (pH = 12) in einem Volumenverhältnis von 4 : 1 (Verhältnis von organischer Phase zu wässriger Phase) kontaktiert (*Extraktion*). Es wurden ein organischer Raffinatstrom und ein wässriger Extraktstrom erhalten. Der wässrige Extraktstrom wurde kontinuierlich durch Zugabe von Schwefelsäure auf einen neutralen pH-Wert gestellt und in einem zweistufigen Mixer-Settler mit einem künstlich erzeugten Hochsiederstrom (enthaltend 50 Massen-% Anilin und 50 Massen-% Diphenylamin als exemplarischen Vertreter typischer Hochsieder eines Anilinprozesses) in einem einem Volumenverhältnis von 2 : 1 (Verhältnis von wässriger Phase zu organischer Phase) kontaktiert (*Säurebehandlung* und *Rückextraktion*). Extraktion und Rückextraktion wurden kontinuierlich und in Reihe bei einer Temperatur von 30 °C durchgeführt. Es wurde gefunden, dass Phenol und Aminophenol im Anilin-Raffinat aus der Extraktion nicht mehr nachweisbar waren. Dies bestätigt die Prinzipversuche auch für den Fall kontinuierlicher Prozessführung. Bei der Rückextraktion wurden im Hochsiederextrakt Phenol und Aminophenol mit hohen Konzentrationen gefunden. Über den gesamten Aufarbeitungsprozess betrachtet wurde gefunden, dass im Mittel 85 % des Phenols und 95 % des Aminophenols aus dem Rohanilinstrom in den Rückextraktionsstrom (in den Hochsiederextrakt) überführt werden konnten. Die Phasentrennung verlief in allen Abscheidern unproblematisch, wobei durch die Zugabe des Salzes Na₂S0₄ die wässrige Phase in allen Abscheidern als schwere Phase erhalten wurde.

## Patentansprüche

1. Verfahren zur Reinigung von Anilin, umfassend die folgenden Schritte:
a) Bereitstellen einer Rohanilinfraktion enthaltend Anilin und organische Verunreinigungen, wobei die organischen Verunreinigungen Phenol, leichter als Anilin siedende organische Verunreinigungen und Aminophenol sowie weitere höher als Anilin siedende organische Verunreinigungen umfassen und die auf die Gesamtmasse der organischen Bestandteile der Rohanilinfraktion bezogene Konzentration an Aminophenol im Bereich von 0,001 Massen-% bis 1,00 Massen-% liegt;
b) Extrahieren der Rohanilinfraktion aus Schritt a) mit einem wässrigen Extraktionsmittel enthaltend, bezogen auf die Gesamtmasse des wässrigen Extraktionsmittels, ein Alkalimetallhydroxid in einem Konzentrationsbereich von 0,009 Massen-% bis 2,05 Massen-% und ein von einem Alkalimetallhydroxid verschiedenes Alkalimetallsalz in einem Konzentrationsbereich von 2,40 Massen-% bis 25,0 Massen-%, wobei nach Phasentrennung eine organische, an Aminophenol abgereicherte, Anilinphase und eine wässrige Aminophenolatphase erhalten werden; und
c) Destillieren der organischen Anilinphase aus Schritt b) unter Erhalt eines Stroms gereinigten Anilins, einem leichter als Anilin siedende organische Verunreinigungen enthaltenden gasförmigen Strom und einem höher als Anilin siedende organische Verunreinigungen und Anilin enthaltenden flüssigen Strom.

2. Verfahren nach Anspruch 1, umfassend die Schritte:
d.1) Aufkonzentrieren des höher als Anilin siedende organische Verunreinigungen und Anilin enthaltenden flüssigen Stroms aus Schritt c) durch Verdampfung von Anilin, wobei ein Anilin-haltiger Destillatstrom und ein höher als Anilin siedende organische Verunreinigungen enthaltender Sumpfstrom erhalten werden;
e.1) Behandeln der in Schritt b) erhaltenen wässrigen Aminophenolatphase mit Säure zur Überführung von Aminophenolat in Aminophenol; und
f.1) Extrahieren von durch die Säurebehandlung gebildetem Aminophenol mit einem organischen Strom umfassend den höher als Anilin siedende organische Verunreinigungen enthaltenden Sumpfstrom aus Schritt d.1), wobei nach Phasentrennung eine organische Phase und eine wässrige Phase erhalten werden, wobei die wässrige Phase mit Alkalimetallhydroxid versetzt und als Bestandteil des in Schritt a) einzusetzenden Extraktionsmittels verwendet wird;

3. Verfahren nach Anspruch 1, umfassend die Schritte:
d.2) Behandeln der in Schritt b) erhaltenen wässrigen Aminophenolatphase mit Säure zur Überführung von Aminophenolat in Aminophenol;
e.2) Extrahieren von durch die Säurebehandlung gebildetem Aminophenol mit einem organischen Strom umfassend den höher als Anilin siedende organische Verunreinigungen und Anilin enthaltenden flüssigen Strom aus Schritt c), wobei nach Phasentrennung eine organische Phase und eine wässrige Phase erhalten werden, wobei die wässrige Phase mit Alkalimetallhydroxid versetzt und als Bestandteil des in Schritt a) einzusetzenden Extraktionsmittels verwendet wird; und
f.2) Aufkonzentrieren der organischen Phase aus Schritt e.2) durch Verdampfung von Anilin, wobei ein Anilin-haltiger Destillatstrom und ein höher als Anilin siedende organische Verunreinigungen enthaltender Sumpfstrom erhalten werden.

4. Verfahren nach Anspruch 2 oder 3, bei welchem der Schritt des Extrahierens (Schritt f.1) bzw. Schritt e.2)) parallel zum Schritt des Behandelns mit Säure (Schritt e.1) bzw. Schritt d.2)) durchgeführt wird.

5. Verfahren nach einem der Ansprüche 2 bis 4, bei welchem Schritt c)
eine Destillation in einer Destillationskolonne mit Seitenabzug umfasst, wobei der Destillationskolonne mit Seitenabzug der Strom gereinigten Anilins als Seitenstrom, der leichter als Anilin siedende organische Verunreinigungen enthaltende gasförmige Strom als Kopfstrom und der höher als Anilin siedende organische Verunreinigungen und Anilin enthaltende flüssige Strom als Sumpfstrom entnommen werden;
oder
eine Destillation in zwei hintereinandergeschalteten Destillationskolonnen umfasst, wobei in der ersten Destillationskolonne der leichter als Anilin siedende organische Verunreinigungen enthaltende gasförmige Strom als Kopfstrom entnommen wird, wobei der in der ersten Destillationskolonne erhaltene Sumpfstrom als Zulauf in die zweite Destillationskolonne geführt wird, wobei in der zweiten Destillationskolonne der Strom gereinigten Anilins als Kopfstrom und der höher als Anilin siedende organische Verunreinigungen und Anilin enthaltende flüssige Strom als Sumpfstrom erhalten werden;
oder
eine Destillation in drei Destillationskolonnen umfasst, wobei einer ersten Destillationskolonne ein Wasser und organische Verunreinigungen enthaltener Kopfstrom entnommen wird, der in eine wässrige und eine organische Phase getrennt wird, wobei die organische Phase in eine zweite Destillationskolonne geführt wird, welcher der leichter als Anilin siedende organische Verunreinigungen enthaltende gasförmige Strom als Kopfstrom entnommen wird, und wobei der in der ersten Destillationskolonne erhaltene Sumpfstrom als Zulauf in eine dritte Destillationskolonne geführt wird, wobei in der dritten Destillationskolonne der Strom gereinigten Anilins als Kopfstrom und der höher als Anilin siedende organische Verunreinigungen und Anilin enthaltende flüssige Strom als Sumpfstrom erhalten werden.

6. Verfahren nach Anspruch 5, bei welchem der leichter als Anilin siedende organische Verunreinigungen enthaltende gasförmige Strom partiell verflüssigt, die so erhaltene flüssige Phase, optional nach Abtrennung von gegebenenfalls vorhandenem Wasser, in diejenige Destillationskolonne, welcher der leichter als Anilin siedende organische Verunreinigungen enthaltende gasförmige Strom als Kopfstrom entnommen wurde, zurückgeführt wird, und wobei die nach der partiellen Verflüssigung verbleibende gasförmige Phase aus der Destillation abgeführt wird.

7. Verfahren nach einem der Ansprüche 2 bis 6, bei welchem die in Schritt c) nach der partiellen Verflüssigung verbleibende gasförmige Phase kondensiert und das erhaltene Kondensat als zusätzlicher Bestandteil des in Schritt f.1) bzw. Schritt e.2) einzusetzenden organischen Stroms verwendet wird.

8. Verfahren nach einem der Ansprüche 2 bis 7, bei welchem
• die in Schritt f.1) nach der Phasentrennung erhaltene organische Phase bzw.
• der in Schritt f.2) erhaltene höher als Anilin siedende organische Verunreinigungen enthaltende Sumpfstrom verbrannt wird.

9. Verfahren nach einem der Ansprüche 2 bis 8, bei welchem das von einem Alkalimetallhydroxid verschiedene Alkalimetallsalz (i) ein Alkalimetallchlorid oder (ii) ein Alkalimetallsulfat ist.

10. Verfahren nach Anspruch 9, bei welchem in Schritt e.1) bzw. in Schritt d.2) als Säure im Fall (i) Salzsäure und im Fall (ii) Schwefelsäure eingesetzt wird.

11. Verfahren nach einem der vorstehenden Ansprüche, bei dem die Rohanilinfraktion durch Hydrierung von Nitrobenzol in Gegenwart eines Katalysators und Abtrennung von in der Hydrierung gebildetem Wasser erhalten wird.

12. Verfahren nach Anspruch 11, bei welchem die Hydrierung von Nitrobenzol in der Gasphase durchgeführt und das erhaltene gasförmige Reaktionsprodukt kondensiert wird,
(i) wobei die Kondensation des gasförmigen Reaktionsprodukts in zwei Stufen mit sukzessive fallender Temperatur durchgeführt wird und nur nach der zweiten Kondensationsstufe eine Phasentrennung zur Abtrennung von in der Hydrierung gebildetem Wasser erfolgt,
oder
(ii) wobei die Kondensation des gasförmigen Reaktionsprodukts einstufig durchgeführt wird und im Anschluss an die Kondensation eine Phasentrennung zur Abtrennung von in der Hydrierung gebildetem Wasser erfolgt, und wobei die nach Abtrennung des Wassers verbleibende organische Phase als Rohanilinstrom in Schritt a) eingesetzt wird.

13. Verfahren nach Anspruch 12, umfassend den Fall (i), bei welchem
(i-1) das in der ersten Kondensationsstufe erhaltene Kondensat mit der nach der Abtrennung von Wasser in der Phasentrennung nach der zweiten Kondensationsstufe verbleibenden organischen Phase vereinigt und als Rohanilinstrom in Schritt a) eingesetzt wird,
oder
(i-2) der in Schritt a) bereitzustellende Rohanilinstrom entweder dem in der ersten Kondensationsstufe erhaltenen Kondensat oder der nach der Abtrennung von Wasser in der Phasentrennung nach der zweiten Kondensationsstufe verbleibenden organischen Phase entnommen wird.

14. Verfahren nach einem der vorstehenden Ansprüche, bei welchem das Alkalimetall des Alkalimetallhydroxids und des von einem Alkalimetallhydroxid verschiedenen Alkalimetallsalzes jeweils Natrium oder Kalium ist.

15. Verfahren nach Anspruch 14, bei welchem
Natrium- oder Kaliumhydroxid in einem Konzentrationsbereich von 0,009 Massen-% bis 2,05 Massen-%
als Alkalimetallhydroxid eingesetzt wird, wobei entweder
Natrium- oder Kaliumchlorid in einem Konzentrationsbereich von 4,00 Massen-% bis 25,0 Massen-%,
oder
Natrium- oder Kaliumsulfat in einem Konzentrationsbereich von 2,40 Massen-% bis 25,0 Massen-%,
als von dem Alkalimetallhydroxid verschiedenen Alkalimetallsalz eingesetzt wird.

## Claims

1. Process for the purification of aniline, comprising the following steps:
a) providing a crude aniline fraction containing aniline and organic impurities, wherein the organic impurities comprise phenol, organic impurities having a lower boiling point than aniline, and aminophenol, and also further organic impurities having a higher boiling point than aniline, and the concentration of aminophenol, based on the total mass of the organic constituents of the crude aniline fraction, is in the range from 0.001% by mass to 1.00% by mass;
b) extracting the crude aniline fraction from step a) with an aqueous extractant containing, based on the total mass of the aqueous extractant, an alkali metal hydroxide in a concentration range from 0.009% by mass to 2.05% by mass and an alkali metal salt different from an alkali metal hydroxide in a concentration range from 2.40% by mass to 25.0% by mass,
wherein, after phase separation, an organic aniline phase depleted in aminophenol and an aqueous aminophenolate phase are obtained; and
c) distilling the organic aniline phase from step b) to obtain a stream of purified aniline, a gaseous stream containing organic impurities having a lower boiling point than aniline, and a liquid stream containing aniline and organic impurities having a higher boiling point than aniline.

2. Process according to Claim 1, comprising the steps of:
d.1) concentrating the liquid stream from step c) containing aniline and organic impurities having a higher boiling point than aniline by evaporation of aniline to obtain an aniline-containing distillate stream and a bottom stream containing organic impurities having a higher boiling point than aniline;
e.1) treating the aqueous aminophenolate phase obtained in step b) with acid to convert aminophenolate to aminophenol; and
f.1) extracting aminophenol formed by the acid treatment with an organic stream comprising the bottom stream from step d.1) containing organic impurities having a higher boiling point than aniline, to obtain, after phase separation, an organic phase and an aqueous phase, wherein alkali metal hydroxide is added to the aqueous phase and this aqueous phase is used as a constituent of the extractant to be used in step a).

3. Process according to Claim 1, comprising the steps of:
d.2) treating the aqueous aminophenolate phase obtained in step b) with acid to convert aminophenolate to aminophenol;
e.2) extracting aminophenol formed by the acid treatment with an organic stream comprising the liquid stream from step c) containing aniline and organic impurities having a higher boiling point than aniline, to obtain, after phase separation, an organic phase and an aqueous phase, wherein alkali metal hydroxide is added to the aqueous phase and this aqueous phase is used as a constituent of the extractant to be used in step a); and
f.2) concentrating the organic phase from step e.2) by evaporation of aniline to obtain an aniline-containing distillate stream and a bottom stream containing organic impurities having a higher boiling point than aniline.

4. Process according to Claim 2 or 3, in which the extraction step (step f.1) or step e.2)) is conducted in parallel with the acid treatment step (step e.1) or step d.2)).

5. Process according to any of Claims 2 to 4, in which step c)
comprises a distillation in a distillation column with side draw, wherein the stream of purified aniline is withdrawn as side stream, the gaseous stream containing organic impurities having a lower boiling point than aniline is withdrawn as top stream, and the liquid stream containing aniline and organic impurities having a higher boiling point than aniline is withdrawn as bottom stream from the distillation column with side draw;
or
comprises a distillation in two distillation columns connected in series, wherein in the first distillation column the gaseous stream containing organic impurities having a lower boiling point than aniline is withdrawn as top stream, wherein the bottom stream obtained in the first distillation column is conducted as feed into the second distillation column, wherein in the second distillation column the stream of purified aniline is obtained as top stream and the liquid stream containing aniline and organic impurities having a higher boiling point than aniline is obtained as bottom stream;
or
comprises a distillation in three distillation columns, wherein a top stream containing water and organic impurities is withdrawn from a first distillation column and is separated into an aqueous and an organic phase, the organic phase being conducted into a second distillation column from which the gaseous stream containing organic impurities having a lower boiling point than aniline is withdrawn as top stream, and wherein the bottom stream obtained in the first distillation column is conducted as feed into a third distillation column, wherein in the third distillation column the stream of purified aniline is obtained as top stream and the liquid stream containing aniline and organic impurities having a higher boiling point than aniline is obtained as bottom stream.

6. Process according to Claim 5, in which the gaseous stream containing organic impurities having a lower boiling point than aniline is partially liquefied, the liquid phase thus obtained, optionally after removal of any water present, is recycled into that distillation column from which the gaseous stream containing organic impurities having a lower boiling point than aniline was withdrawn as top stream, and wherein the gaseous phase remaining after the partial liquefaction is discharged from the distillation.

7. Process according to any of Claims 2 to 6, in which the gaseous phase remaining in step c) after the partial liquefaction is condensed and the condensate obtained is used as an additional constituent of the organic stream to be used in step f.1) or step e.2).

8. Process according to any of Claims 2 to 7, in which
• the organic phase obtained in step f.1) after the phase separation or
• the bottom stream obtained in step f.2) and containing organic impurities having a higher boiling point than aniline is incinerated.

9. Process according to any of Claims 2 to 8, in which the alkali metal salt different from an alkali metal hydroxide is (i) an alkali metal chloride or (ii) an alkali metal sulfate.

10. Process according to Claim 9, in which the acid used in step e.1) or in step d.2) in case (i) is hydrochloric acid and in case (ii) is sulfuric acid.

11. Process according to any of the preceding claims, in which the crude aniline fraction is obtained by hydrogenation of nitrobenzene in the presence of a catalyst and removal of water formed in the hydrogenation.

12. Process according to Claim 11, in which the hydrogenation of nitrobenzene is conducted in the gas phase and the gaseous reaction product obtained is condensed,
(i) wherein the condensation of the gaseous reaction product is conducted in two stages with successively falling temperature and only after the second condensation stage a phase separation for removing water formed in the hydrogenation takes place,
or
(ii) wherein the condensation of the gaseous reaction product is conducted in one stage and following the condensation a phase separation for removing water formed in the hydrogenation takes place, and wherein the organic phase remaining after removal of the water is used as crude aniline stream in step a).

13. Process according to Claim 12, comprising case (i), in which
(i-1) the condensate obtained in the first condensation stage is combined with the organic phase remaining after the removal of water in the phase separation after the second condensation stage and is used as crude aniline stream in step a),
or
(i-2) the crude aniline stream to be provided in step a) is withdrawn either from the condensate obtained in the first condensation stage or from the organic phase remaining after the removal of water in the phase separation after the second condensation stage.

14. Process according to any one of the preceding claims, in which the alkali metal of the alkali metal hydroxide and of the alkali metal salt different from an alkali metal hydroxide is in each case sodium or potassium.

15. Process according to Claim 14, in which
the alkali metal hydroxide used is sodium or potassium hydroxide in a concentration range from 0.009% by mass to 2.05% by mass,
wherein the alkali metal salt used which is different from the alkali metal hydroxide is either
sodium or potassium chloride in a concentration range from 4.00% by mass to 25.0% by mass,
or
sodium or potassium sulfate in a concentration range from 2.40% by mass to 25.0% by mass.

## Revendications

1. Procédé de purification d'aniline, comprenant les étapes suivantes :
a) fourniture d'une fraction d'aniline brute contenant de l'aniline et des impuretés organiques, les impuretés organiques comprenant du phénol, des impuretés organiques à point d'ébullition inférieur à celui de l'aniline et de l'aminophénol, ainsi que d'autres impuretés organiques à point d'ébullition supérieur à celui de l'aniline, et la concentration de l'aminophénol, rapportée à la masse totale des constituants organiques de la fraction d'aniline brute, étant comprise dans la plage de 0,001 % en masse à 1,00 % en masse ;
b) extraction de la fraction d'aniline brute de l'étape a) avec un agent d'extraction aqueux contenant, par rapport à la masse totale de l'agent d'extraction aqueux, un hydroxyde d'un métal alcalin dans une plage de concentrations de 0,009 % en masse à 2,05 % en masse et un sel d'un métal alcalin, différent d'un hydroxyde d'un métal alcalin, dans une plage de concentrations de 2,40 % en masse à 25,0 % en masse, avec obtention d'une phase aniline organique appauvrie en aminophénol et d'une phase aminophénolate aqueuse après la séparation des phases ; et
c) distillation de la phase aniline organique de l'étape b) avec obtention d'un courant d'aniline purifiée, d'un courant gazeux contenant des composés organiques à point d'ébullition inférieur à celui de l'aniline et un courant liquide contenant de l'aniline et des impuretés organiques à point d'ébullition supérieur à celui de l'aniline.

2. Procédé selon la revendication 1, comprenant les étapes :
d.1) concentration du courant liquide contenant de l'aniline et des composés organiques à point d'ébullition supérieur à celui de l'aniline de l'étape c) par évaporation de l'aniline, avec obtention d'un courant de distillat contenant de l'aniline et d'un courant de fond contenant des impuretés organiques à point d'ébullition supérieur à celui de l'aniline ;
e.1) traitement de la phase aminophénolate aqueuse obtenue dans l'étape b) avec un acide pour conversion de l'aminophénolate en aminophénol ; et
f.1) extraction de l'aminophénol formé par le traitement à l'acide, avec un courant organique comprenant le courant de fond contenant des impuretés organiques à point d'ébullition supérieur à celui de l'aniline de l'étape d.1), avec, après la séparation des phases, obtention d'une phase organique et d'une phase aqueuse, la phase aqueuse étant additionnée de l'hydroxyde d'un métal alcalin et étant utilisée comme constituant de l'agent d'extraction à utiliser dans l'étape a) .

3. Procédé selon la revendication 1, comprenant les étapes :
d.2) traitement de la phase aminophénolate aqueuse obtenue dans l'étape b) avec un acide pour conversion de l'aminophénolate en aminophénol ;
e.2) extraction de l'aminophénol formé par le traitement à l'acide avec un courant organique comprenant le courant liquide contenant les impuretés organiques à point d'ébullition supérieur à celui de l'aniline et de l'aniline de l'étape c), avec, après la séparation des phases, obtention d'une phase organique et d'une phase aqueuse, la phase aqueuse étant additionnée de l'hydroxyde d'un métal alcalin et étant utilisée comme constituant de l'agent d'extraction à utiliser dans l'étape a) ; et
f.2) concentration de la phase organique de l'étape e.2) par évaporation de l'aniline, avec obtention d'un courant de distillat contenant de l'aniline et d'un courant de fond contenant des impuretés organiques à point d'ébullition supérieur à celui de l'aniline.

4. Procédé selon la revendication 2 ou 3, dans lequel l'étape d'extraction (étape f.1) ou étape e.2)) est mise en œuvre parallèlement à l'étape de traitement à l'acide (étape e.1) ou étape d.2)).

5. Procédé selon l'une des revendications 2 à 4, dans lequel l'étape c) comprend une distillation dans une colonne de distillation avec soutirage latéral, dans lequel on prélève de la colonne de distillation avec soutirage latéral le courant d'aniline purifiée en tant que courant latéral, le courant gazeux contenant des impuretés organiques à point d'ébullition inférieur à celui de l'aniline en tant que courant de tête, et, en tant que courant de fond, le courant liquide contenant de l'aniline et des impuretés organiques à point d'ébullition supérieur à l'aniline ;
ou
comprend une distillation dans deux colonnes de distillation successives, le premier courant gazeux contenant des impuretés organiques à point d'ébullition inférieur à celui de l'aniline étant prélevé en tant que courant de tête dans la première colonne de distillation, le courant de fond obtenu dans la première colonne de distillation étant envoyé en tant qu'alimentation dans la deuxième colonne de distillation, pour obtenir dans la deuxième colonne de distillation le courant d'aniline purifiée en tant que courant de tête, et, en tant que courant de fond, le courant liquide contenant de l'aniline et des impuretés organiques à point d'ébullition supérieur à celui de l'aniline ;
ou
comprend une distillation dans trois colonnes de distillation, un courant de tête contenant de l'eau et des impuretés organiques étant prélevé d'une première colonne de distillation, ce courant étant séparé en une phase aqueuse et une phase organique, la phase organique étant envoyée dans une deuxième colonne de distillation, dont on prélève en tant que courant de tête le courant gazeux contenant des impuretés organiques à point d'ébullition inférieur à celui de l'aniline, et le courant de fond obtenu dans la première colonne de distillation étant envoyé en tant qu'alimentation dans une troisième colonne de distillation, avec dans la troisième colonne de distillation obtention du courant d'aniline purifiée en tant que courant de tête, et en tant que courant de fond le courant liquide contenant de l'aniline et des impuretés organiques à point d'ébullition supérieur à celui de l'aniline.

6. Procédé selon la revendication 5, dans lequel le courant gazeux contenant des impuretés organiques à point d'ébullition inférieur à celui de l'aniline est partiellement liquéfié, la phase liquide obtenue, éventuellement après séparation de l'eau éventuellement présente, étant renvoyée dans la colonne de distillation dont avait été prélevé en tant que courant de tête le courant gazeux contenant des impuretés organiques à point d'ébullition inférieur à celui de l'aniline, et la phase gazeuse restant après la liquéfaction partielle étant évacuée de la distillation.

7. Procédé selon l'une des revendications 2 à 6, dans lequel la phase gazeuse restant dans l'étape c) après la liquéfaction partielle est condensée, et le condensat obtenu est utilisé en tant que constituant supplémentaire du courant organique à utiliser dans l'étape f.1) ou dans l'étape e.2).

8. Procédé selon l'une des revendications 2 à 7, dans lequel on brûle
• la phase organique obtenue dans l'étape f.1) après la séparation des phases ou
• le courant de fond obtenu dans l'étape f.2), contenant des impuretés organiques à point d'ébullition supérieur à celui de l'aniline.

9. Procédé selon l'une des revendications 2 à 8, dans lequel le sel d'un métal alcalin différent de l'hydroxyde d'un métal alcalin est (i) le chlorure d'un métal alcalin ou (ii) le sulfate d'un métal alcalin.

10. Procédé selon la revendication 9, dans lequel on utilise dans l'étape e.1) ou dans l'étape d.2) en tant qu'acide dans le cas (i) de l'acide chlorhydrique et dans le cas (ii) de l'acide sulfurique.

11. Procédé selon l'une des revendications précédentes, dans lequel la fraction d'aniline brute est obtenue par hydrogénation d'un nitrobenzène en présence d'un catalyseur et séparation de l'eau formée lors de l'hydrogénation.

12. Procédé selon la revendication 11, dans lequel l'hydrogénation du nitrobenzène est réalisée en phase gazeuse, et le produit de réaction gazeux obtenu est condensé,
(i) la condensation du produit de réaction gazeux étant mise en œuvre en deux étapes à des températures successives décroissantes, une séparation des phases n'ayant lieu qu'après la deuxième étape de condensation pour séparer l'eau formée lors de l'hydrogénation,
ou
(ii) la condensation du produit de réaction gazeux étant mise en œuvre en une étape, une séparation des phases ayant lieu après la condensation pour séparer l'eau formée lors de l'hydrogénation, et la phase organique restant après séparation de l'eau étant utilisée en tant que courant d'aniline brute dans l'étape a).

13. Procédé selon la revendication 12, comprenant le cas (i), dans lequel
(i-1) on purifie le condensat obtenu dans la première étape de condensation avec la phase organique restant après séparation de l'eau lors de la séparation des phases après la deuxième étape de condensation, et on l'utilise en tant que courant d'aniline brute dans l'étape a),
ou
(i-2) on prélève le courant d'aniline brute à préparer dans l'étape a) soit du condensat obtenu dans la première étape de condensation, soit de la phase organique restant après la séparation de l'eau lors de la séparation des phases après la deuxième étape de condensation.

14. Procédé selon l'une des revendications précédentes, dans lequel le métal alcalin de l'hydroxyde d'un métal alcalin et du sel d'un métal alcalin différent de l'hydroxyde d'un métal alcalin est dans chaque cas le sodium ou le potassium.

15. Procédé selon la revendication 14, dans lequel
on utilise en tant qu'hydroxyde d'un métal alcalin de l'hydroxyde de sodium ou de potassium dans une plage de concentrations de 0,009 % en masse à 2,05 % en masse,
auquel cas on utilise en tant que sel d'un métal alcalin différent de l'hydroxyde d'un métal alcalin du chlorure de sodium ou de potassium dans une plage de concentrations de 4,00 % en masse à 25,0 % en masse ou
du sulfate de sodium ou du sulfate de potassium dans une plage de concentrations de 2,40 % en masse à 25,0 % en masse.
